(19) Europäisches Patentamt
European Patent Office
Office européen des brevets

(11) EP 1 013 649 A1

# (12) EUROPEAN PATENT APPLICATION

(43) Date of publication:
**28.06.2000 Bulletin 2000/26**

(51) Int. Cl.[7]: **C07D 307/36**, C09K 19/34, C09K 19/42, C09K 19/46

(21) Application number: **99125327.9**

(22) Date of filing: **20.12.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **22.12.1998 US 113506 P**

(71) Applicant: **CHISSO CORPORATION**
**Osaka-shi Osaka 530-6591 (JP)**

(72) Inventors:
- **Bernhardt, Henry**
**13158 Berlin (DE)**
- **Kato, Takashi**
**Ichihara-shi, Chiba-ken (JP)**
- **Fujita, Atsuko**
**Chiba-shi, Chiba-ken (JP)**
- **Takeuchi, Hiroyuki**
**Ichihara-shi, Chiba-ken (JP)**
- **Takeshita, Fusayuki**
**Sodegaura-shi, Chiba-ken (JP)**
- **Nakagawa, Etsuo**
**Ichihara-shi, Chiba-ken (JP)**

(74) Representative: **HOFFMANN - EITLE**
**Patent- und Rechtsanwälte**
**Arabellastrasse 4**
**81925 München (DE)**

## (54) Liquid crystal compounds incorporating furan ring, liquid crystal composition and liquid crystal display device

(57) The present invention provides a furan derivative compound having physical properties suitable as a liquid crystal display material, for example, such as 5-(4'-trans-pentyl-trans-bicyclohexyl-4-yl)-2-methylfuran, 5-(4-(4-trans-pentylcyclohexyl)phenyl)-2-ethylfuran, 5-(trans-4-propylcyclohexyl)fur-2-yl-3,4-dibromobenzene, 2-(4-trans-pentylcyclohexyl)-5-(4-trans-propylcyclohexyl)furan and 2-(4-trans-(2,3-difluoro-4-methoxyphenyl)cyclohexyl)-5-(5-trans-propyl-1,3-dioxan-2-yl)furan; a liquid crystal composition containing the said compound; and a liquid crystal display device comprising the said liquid crystal composition.

EP 1 013 649 A1

## Description

Technical field

This invention relates to liquid crystalline compounds incorporating a furan ring which are useful as a liquid crystal display material. More specifically, the present invention relates to a novel compound which has a furan-2,5-diyl group and which can express some physical properties suitable as a liquid crystal display material, a liquid crystal composition containing the said compound, and a liquid crystal display devices comprising the said liquid crystal composition.

Background art

Devices employing liquid crystals are used in a variety of electrooptical applications, in particular those which require compact, energy efficient, voltage controlled light valves, e.g., watch and calculator displays, as well as flat panel displays used in portable computers and compact televisions. Unique characteristics of liquid crystal displays including low operation voltage and low power consumption make them the most promising candidates of non-emissive electrooptical display currently available.

Electrooptical devices employing liquid crystals can be based on various effects. Examples of such devices are cells having dynamic scattering (DS), deformation of aligned phases (DAP) cells, cells based on guest host effect (GH), twisted nematic (TN) cells, supertwisted nematic (STN) cells, superbirefringence effect (SBE) cells and optical mode interference (OMI) cells. Most common display devices have a twisted nematic structure and are based on the Schadt-Helfrich effect. The demand for liquid crystal display devices is increasing year by year. In particular, the coloring of the liquid crystal displays has remarkably progressed in recent years. In the case of display systems for the color display, a thin film transistor type (TFT type) and a supertwisted nematic type (STN type) are mainly used. Display devices having these display systems have been mass-produced.

Liquid crystal materials for the use in electrooptical devices must have good chemical and thermal stability and good stability against electric fields. Short addressing times, relatively low viscosity, low threshold voltages and high contrast in the cells are other required properties. The liquid crystal material must have a suitable mesophase at the operating temperature for the mentioned cells.

To optimize the properties of a liquid crystal material, liquid crystal mixtures are generally used. Therefore, good miscibility of the components is necessary. The properties of a liquid crystal mixture such as dielectrical and optical anisotropy and conductivity must satisfy the requirements of the cell type and the requirements of the application. For example, materials for cells with twisted nematic structure should have a low conductivity and positive dielectric anisotropy. It is also important that the liquid crystal material is stable against heating and exposure to UV radiation. The temperature dependence of the viscosity should be very small and crystallization should not occur even at very low temperatures. Also other properties of the liquid crystal material such as clearing point, melting point, viscosity, dielectric quantities, elastic quantities and birefringence have to be optimized. An improvement of the properties of liquid crystal compounds for the applications given above is urgently desired.

Currently used liquid crystal compositions still have limitations regarding the application area. In most cases the low temperature behavior of the liquid crystal material is not suitable. So display devices for very low temperature ranges are not available until now. For the most common applications like TFT type and STN type the viscosity of the liquid crystal material limits the speed of the display. There is also an interest in compounds with negative dielectric anisotropy for applications based on electrically controlled birefringence (ECB) or positive-contrast guest-host (GH) technique. The recent liquid crystal materials for these applications exhibit relatively high viscosity, leading to long switching times.

Because of these limitations on different applications, there is a demand for materials having improved low temperature behavior and low viscosity, and also large positive or large negative dielectric anisotropy in dependence on the application.

Heretofore, few furan derivatives are known as liquid crystal materials. Most of these compounds contain a 2-furyl unit as terminal group. These compounds are chemically unstable and do not have any advantage for application in a liquid crystal display device. Another class of furan containing liquid crystals are esters of substituted furan-2-carboxylic acid, for example, compound A mentioned in Japanese Patent Kokai No. 63-60981:

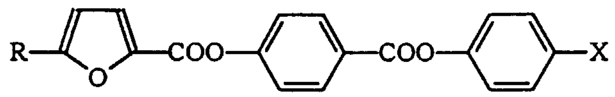

A

wherein R is an alkyl chain and X is selected from the group consisting of -CN, -F, -Cl and -Br. These derivatives are known to have a very high viscosity and small range of the liquid crystal phase.

Disclosure of the invention

The present invention has been intended to solve the problems of conventional techniques, and an object of the present invention is to provide a liquid crystal compound having a particularly wide liquid crystal phase temperature range, a liquid crystal phase at low temperature, an improved solubility at low temperature, a low viscosity, a liquid crystal composition containing this liquid crystal compound, and a liquid crystal display device comprising the liquid crystal composition.

The inventors have intensively researched on a compound which can solve the problems, and as a result, it has been found that a liquid crystal compound containing a furan-2,5-diyl moiety possesses a wide liquid crystal temperature range, a liquid crystal phase at low temperature, a high solubility at low temperature and a low viscosity. Thus, the inventors have completed the present invention on the basis of this knowledge.

The aspects of the present invention are as follows:

1. A liquid crystal compound incorporating a furan-2,5-diyl moiety which has nematic, smectic, latent nematic or smectic mesophases and is represented by the formula (1)

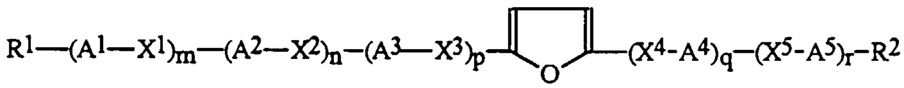

(1)

where $A^1$, $A^2$, $A^3$, $A^4$ and $A^5$ are each independently trans-1,4-cyclohexylene in which one or two methylene group(s) may optionally be substituted with difluoromethylene group(s), 1,4-cyclohexenylene, 1,3-dioxane-2,5-diyl, tetrahydropyran-2,5-diyl in which one methylene group may optionally be substituted with a difluoromethylene group, 1,3-dithiane-2,5-diyl, 1,4-phenylene in which one or more hydrogen atom(s) may optionally be substituted with halogen atom(s) or $-CF_3$ group(s), pyrimidine-2,5-diyl, pyridine-2,5-diyl in which one hydrogen atom may optionally be substituted with a halogen atom, thiazole-2,5-diyl, thiadiazole-2,5-diyl or thiophene-2,5-diyl; $X^1$, $X^2$, $X^3$, $X^4$ and $X^5$ are each independently $-(CH_2)_2-$ or $-(CH_2)_4-$ where one $-CH_2-$ group may be substituted with -O-, -S-, -CH=CH-, -C≡C-, $-CF_2O-$, $-OCF_2-$, $-CF_2S-$ or $-SCF_2-$, or a single bond; m, n, p, q and r are each independently an integer from 0 to 2 with the proviso that $1 \le m + n + p + q + r \le 4$; and $R^1$ and $R^2$ are each independently a straight or branched alkyl chain of 1 to 18 carbon atoms in which one or more $-CH_2-$ group(s) may be substituted with -O-, -S-, $-CF_2-$, -CHF-, $-SiH_2-$, $-SiH(CH_3)-$, $-Si(CH_3)_2-$, - CH=CH- or -C≡C- with the proviso that two similar heteroatoms are not neighbored, and the terminal $-CH_3$ group may be substituted with $-CF_3$, $-OCF_3$, $-CF_2H$, $-CH=CH_2$, $-CH=CF_2$, -CN or a halogen atom; provided that $R^1$ shall not be -CN if q = 1, m = n = p = r = 0 , $X^4$ is a single bond, $A^4$ is 1,4-phenylene and $R^2$ is an n-alkyl chain, and that $R^2$ shall not be -CN if p = 1, m = p = q = r = 0 , $X^3$ is a single bond, $A^3$ is 1,4-phenylene and $R^1$ is an n-alkyl chain.

2. A liquid crystal compound of item 1 represented by the formula (1); where $A^1$, $A^2$, $A^3$, $A^4$ and $A^5$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$, $X^2$, $X^3$, $X^4$ and $X^5$ are each independently - $(CH_2)_2-$ or $-(CH_2)_4-$ in which one $-CH_2-$ group may be substituted with -O-, -C≡C-, $-CF_2O-$ or $-OCF_2-$, or a single bond; m, n, p, q and r are each independently an integer from 0 to 2 with the proviso that $1 \le m + n + p + q + r \le 4$; and $R^1$ and $R^2$ are each independently a straight or branched alkyl chain of 1 to 12 carbon atoms in which one or more - $CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

3. A liquid crystal compound of item 1 represented by the formula (1); where $A^1$, $A^2$ and $A^5$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$, $X^2$ and $X^5$ are each independently $-(CH_2)_2-$ or $-(CH_2)_4-$in which one $-CH_2-$ group may be substituted with -O-, -C≡C-, $-CF_2O-$ or $-OCF_2-$, or a single bond; m, n and r are each independently an integer from 0 to 2 with the proviso that $m + n + r = 3$ and $p = q = 0$; and $R^1$ and $R^2$ are each independently a straight or branched alkyl chain of 1 to 12 carbon atoms in which one or more $-CH_2-$

group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

4. A liquid crystal compound of item 1 represented by the formula (1); where $A^1$, $A^2$ and $A^3$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$, $X^2$ and $X^3$ are each independently $-(CH_2)_2-$ or $-(CH_2)_4-$in which one $-CH_2-$ group may be substituted with -O-, -C≡C-, $-CF_2O-$ or $-OCF_2-$, or a single bond; m, n and p are each independently an integer from 0 to 2 with the proviso that $m + n + p = 3$ and $q = r = 0$; and $R^1$ and $R^2$ are each independently a straight or branched alkyl chain of 1 to 12 carbon atoms in which one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

5. A liquid crystal compound of item 1 represented by the formula (1); where $A^1$ and $A^5$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, pyridine-2,5-diyl in which one hydrogen atom may optionally be substituted with a halogen atom; $X^1$ and $X^5$ are each independently $-(CH_2)_2-$ or $-(CH_2)_4-$ where one - $CH_2-$ group may be substituted with -O-, -C≡C-, $-CF_2O-$ or - $OCF_2-$, or a single bond; m and r are each independently an integer from 0 to 2 with the proviso that $m + r = 2$ and $n = p = q = 0$; and $R^1$ and $R^2$ are each independently a straight or branched alkyl chain of 1 to 12 carbon atoms where one or more $-CH_2-$ group(s) may be substituted with -O- or -CH=CH- with the proviso that two oxygen atoms are not neighbored, and the terminal $-CH_3$ group may be substituted with $-CF_3$, - $OCF_3$, $-CF_2H$, $-CH=CH_2$, $-CH=CF_2$, -CN or a halogen atom selected from fluorine and chlorine.

6. A liquid crystal compound of item 1 represented by the formula (1); where $A^1$ and $A^2$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$ and $X^2$ are each independently $-(CH_2)_2-$ or $-(CH_2)_4-$ in which one $-CH_2-$ group may be substituted with -O-, -C≡C-, - $CF_2O-$ or $-OCF_2-$, or a single bond; m and n are each independently an integer from 0 to 2 with the proviso that $m + n = 2$ and $p = q = r = 0$; and $R^1$ and $R^2$ are each independently a straight or branched alkyl chain of 1 to 12 carbon atoms where one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$ or a halogen atom selected from fluorine and chlorine.

7. A liquid crystal compound of item 1 represented by the formula (1); where $A^1$, $A^2$ and $A^3$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $A^4$ is trans-1,4-cyclohexylene; $X^1$, $X^2$ and $X^3$ are each independently $-(CH_2)_2-$ or $-(CH_2)_4-$ where one $-CH_2-$ group may be substituted with -O-, -C≡C-, $-CF_2O-$ or - $OCF_2-$, or a single bond; $X^4$ is a single bond; m, n and p are each independently an integer from 0 to 2 with the proviso that $1 \leq m + n + p \leq 3$, q = 1 and r = 0; and $R^1$ and $R^2$ are each independently a straight or branched alkyl chain of 1 to 12 carbon atoms in which one or more $-CH_2-$ group(s) may be substituted with -O-or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

8. A liquid crystal compound of item 1 represented by the formula (1); where $A^1$ is trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$ is $-(CH_2)_2-$ or $-(CH_2)_4-$where one $-CH_2-$ group may be substituted with -O-, -C≡C-, - $CF_2O-$ or $-OCF_2-$, or a single bond; $m = 1$; $n = p = q = r = 0$; and $R^1$ and $R^2$ are each independently a straight or branched alkyl chain of 1 to 12 carbon atoms in which one or more - $CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

9. A liquid crystal composition which comprises two or more components containing at least one liquid crystal compound described in any one of items 1 to 8.

10. A liquid crystal composition which contains at least one liquid crystal compound described in any one of items 1 to 8 as a first component and at least one compound selected from the group consisting of the formulae (2), (3) and (4) as a second component,

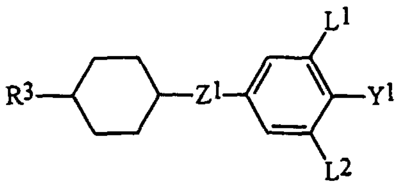

(2)

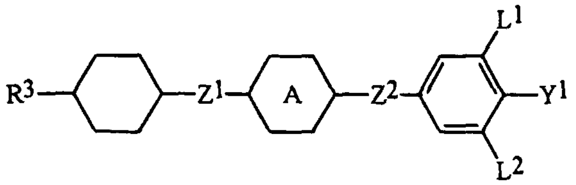

(3)

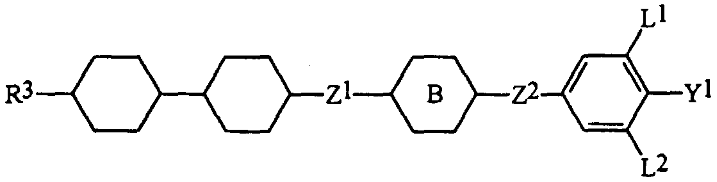

(4)

where $R^3$ is an alkyl group having 1 to 10 carbon atoms in which any unadjacent methylene group may be substituted with an oxygen atom or -CH=CH- and any hydrogen atom may be substituted with a fluorine atom; $Y^1$ is F, Cl, $OCF_3$, $OCF_2H$, $CF_3$, $CF_2H$, $CFH_2$, $OCF_2CF_2H$ or $OCF_2CFHCF_3$; $L^1$ and $L^2$ are each independently H or F; $Z^1$ and $Z^2$ are each independently - $(CH_2)_2$-, -$(CH_2)_4$-, -COO-, -$CF_2O$-, -$OCF_2$-, -CH=CH- or a single bond; ring A is trans-1,4-cyclohexylene, 1,3-dioxane-2,5-diyl or 1,4-phenylene in which a hydrogen atom may be substituted with a halogen atom; and ring B is trans-1,4-cyclohexylene or 1,4-phenylene in which a hydrogen atom may be substituted with a halogen atom.

11. A liquid crystal composition which contains at least one liquid crystal compound described in any one of items 1 to 8 as a first component and at least one compound selected from the group consisting of the formulae (5) and (6) as a second component,

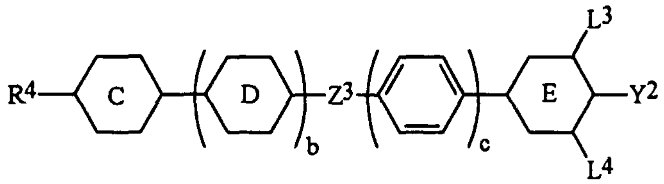

(5)

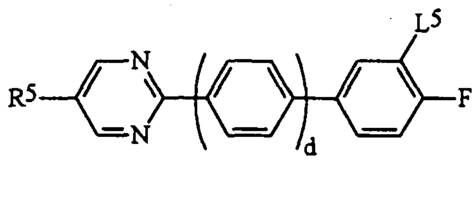

(6)

where $R^4$ and $R^5$ are each independently an alkyl group having 1 to 10 carbon atoms in which any unadjacent methylene group may be substituted with an oxygen atom or -CH=CH- and any hydrogen atom may be substituted with a fluorine atom; $Y^2$ is -CN or -C≡C-CN; ring C is trans-1,4-cyclohexylene, 1,4-phenylene, 1,3-dioxane-2,5-diyl or pyrimidine-2,5-diyl; ring D is trans-1,4-cyclohexylene, 1,4-phenylene in which a hydrogen atom may be substituted with a fluorine atom, or pyrimidine-2,5-diyl; ring E is trans-1,4-cyclohexylene or 1,4-phenylene; $Z^3$ is $-(CH_2)_2-$, -COO- or a single bond; $L^3$, $L^4$ and $L^5$ are each independently a hydrogen atom or a fluorine atom; and b, c and d are each independently 0 or 1.

12. A liquid crystal composition which contains at least one liquid crystal compound described in any one of items 1 to 8 as a first component and at least one compound selected from the group consisting of the formulae (7), (8) and (9) as a second component,

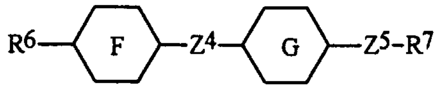

(7)

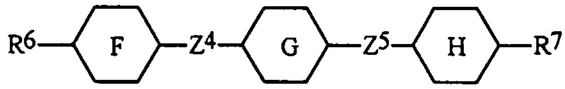

(8)

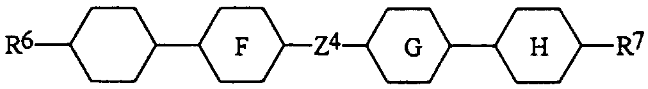

(9)

where $R^6$ and $R^7$ are each independently an alkyl group having 1 to 10 carbon atoms in which any unadjacent methylene group may be substituted with an oxygen atom or -CH=CH- and any hydrogen atom may be substituted with a fluorine atom; rings F, G and H are each independently trans-1,4-cyclohexylene, pyrimidine-2,5-diyl or 1,4-phenylene in which a hydrogen atom may be substituted with a fluorine atom; and $Z^4$ and $Z^5$ are each independently -C≡C-, -COO-, $-(CH_2)_2-$, -CH=CH- or a single bond.

13. A liquid crystal composition which contains at least one liquid crystal compound described in any one of items 1 to 8 as a first component and at least one compound selected from the group consisting of the formulae (10), (11) and (12) as a second component,

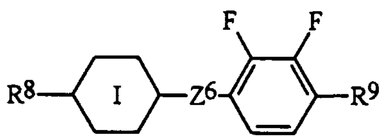

(10)

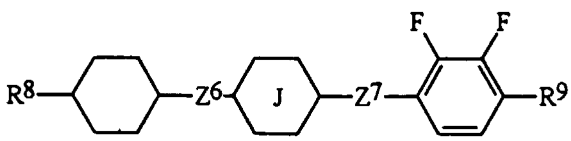

(11)

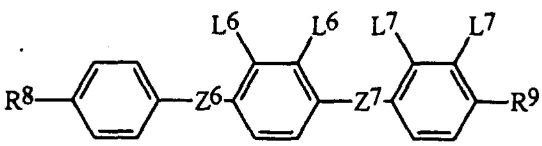

(12)

wherein $R^8$ and $R^9$ are each independently an alkyl group having 1 to 10 carbon atoms or an alkenyl group having 2 to 10 carbon atoms in which any methylene group $(-CH_2-)$ may be substituted with an oxygen atom (-O-) but two methylene groups are not continuously substituted with oxygen atoms and any hydrogen atom in the alkyl group may be substituted with a fluorine atom; ring I and ring J are each independently a trans-1,4-cyclohexylene group or a 1,4-phenylene group; $L^6$ and $L^7$ are each independently a hydrogen atom or a fluorine atom with the proviso

that $L^6$ and $L^7$ cannot be hydrogen atoms simultaneously; and $Z^6$ and $Z^7$ are each independently $-(CH_2)_2-$, $-CH_2O-$ or a single bond.

14. A liquid crystal composition which comprises at least one liquid crystal compound described in any one of items 1 to 8 as a first component, at least one compound selected from the group consisting of the formulae (2), (3) and (4) as a second component and at least one compound selected from the group consisting of the formulae (5) and (6) as a third component.

15. A liquid crystal composition which comprises at least one liquid crystal compound described in any one of items 1 to 8 as a first component, at least one compound selected from the group consisting of the formulae (2), (3) and (4) as a second component and at least one compound selected from the group consisting of the formulae (7), (8) and (9) as a third component.

16. A liquid crystal composition which comprises at least one liquid crystal compound described in any one of items 1 to 8 as a first component, at least one compound selected from the group consisting of the formulae (5) and (6) as a second component and at least one compound selected from the group consisting of the formulae (7), (8) and (9) as a third component.

17. A liquid crystal composition which comprises at least one liquid crystal compound described in any one of items 1 to 8 as a first component, at least one compound selected from the group consisting of the formulae (2), (3) and (4) as a second component, at least one compound selected from the group consisting of the formulae (5) and (6) as a third component and at least one compound selected from the group consisting of the formulae (7), (8) and (9) as a fourth component.

18. A liquid crystal composition which contains at least one liquid crystal composition described in any one of items 9 to 17 of the present invention and at least one optically active compound.

19. A liquid crystal display device which comprises a liquid crystal composition described in any one of items 9 to 18.

The compounds of the present invention are colorless and have a number of desirable properties when used in admixture with other liquid crystal compounds, preferably compounds with nematic mesophases. For example, the compounds of the invention when admixed with such preferred liquid crystal compounds show excellent compatibility, beneficial effect or only a minimal negative effect on the nematic temperature range of the resulting mixtures (even when present in high concentrations), lower viscosity, significantly smaller anisotropy of birefringence, and a good effect on the threshold voltage ratio. The compounds of the invention exhibit high chemical and thermal stability as well as excellent stability against UV radiation in admixture with other liquid crystal compounds. Moreover, in the case that a liquid crystal composition containing the compounds of the present invention is used as constitutional component for a liquid crystal display device, the obtained liquid crystal display device is stable to an external environment and driveable at low voltage, and has a wide use temperature range respectively at low temperature, a high-speed response and a high contrast. In particular, the compounds of the present invention are extremely excellent as constitutional component to adjust the properties of a liquid crystal composition.

Best mode for carrying out the invention

Each furan derivative compound represented by the formula (1) of the present invention exhibits suitable physical properties as a liquid crystal material. By using the compounds of the formula (1) in which $A^1$, $A^2$, $A^3$, $A^4$, $A^5$, $X^1$, $X^2$, $X^3$, $X^4$, $X^5$, m, n, p, q, r, $R^1$ and $R^2$ are suitably selected, a liquid crystal composition suitable for use purpose can be prepared.

Of the compounds of the formula (1), particularly preferable compounds as liquid crystal materials can be represented by the following formulae (1-1) to (1-42):

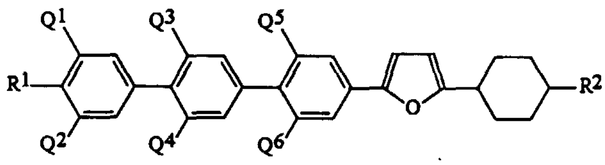

(1-1)

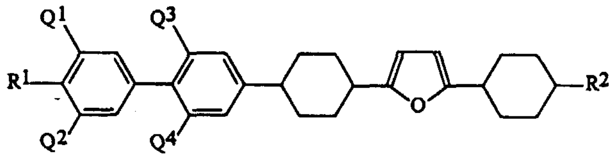

(1-2)

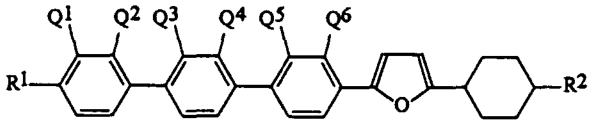

(1-3)

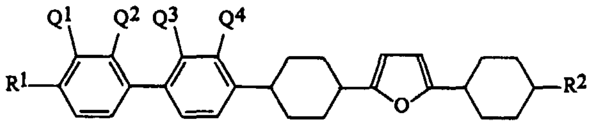

(1-4)

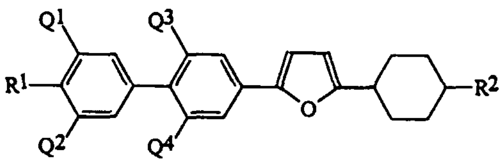

(1-5)

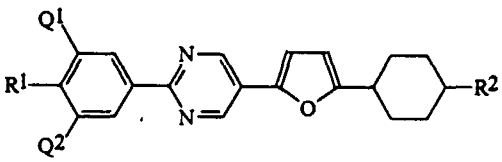

(1-6)

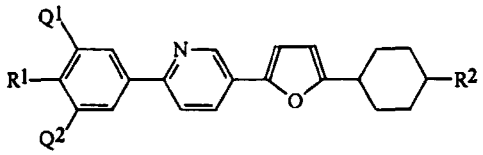

(1-7)

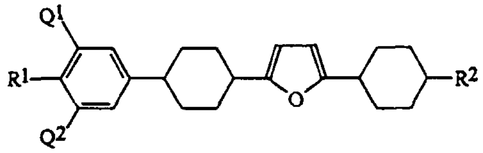

(1-8)

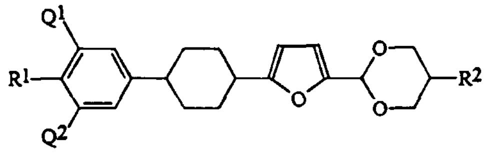

(1-9)

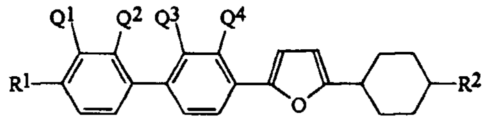

(1-10)

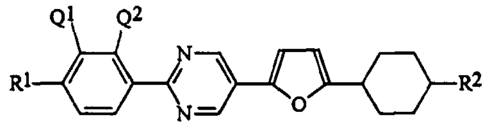

(1-11)

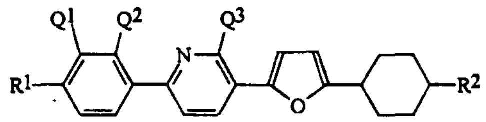

(1-12)

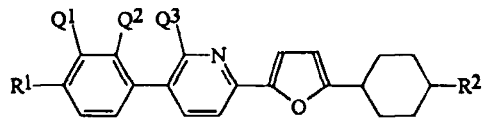

(1-13)

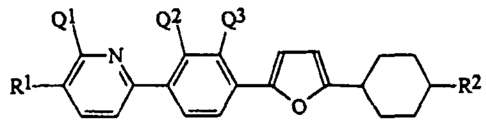

(1-14)

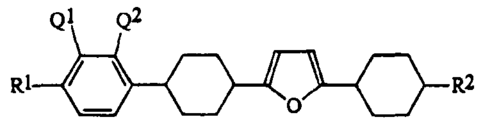

(1-15)

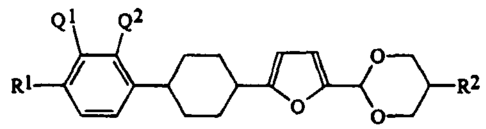

(1-16)

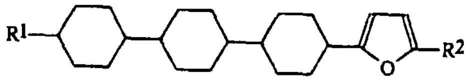

(1-17)

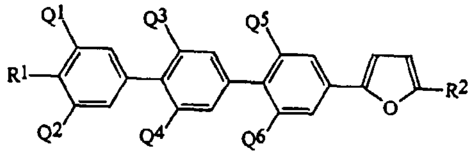

(1-18)

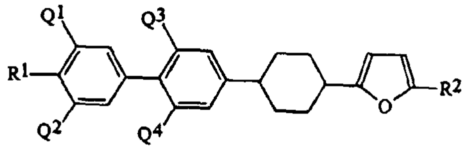

(1-19)

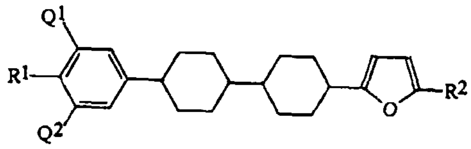

(1-20)

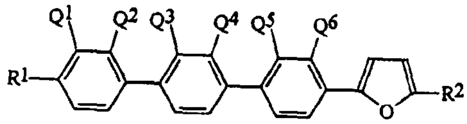

(1-21)

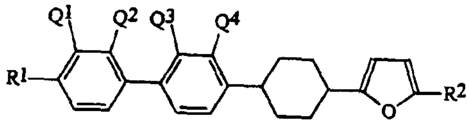

(1-22)

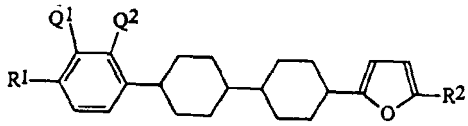

(1-23)

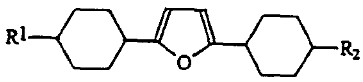

(1-24)

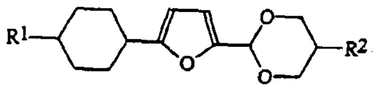

(1-25)

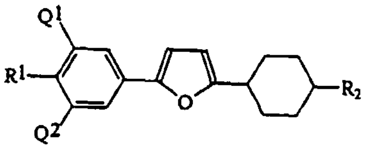

(1-26)

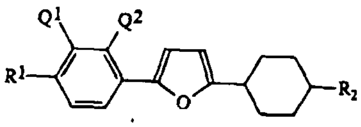

(1-27)

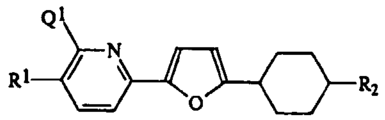

(1-28)

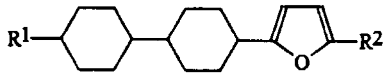

(1-29)

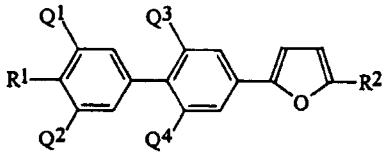

(1-30)

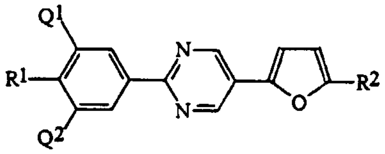

(1-31)

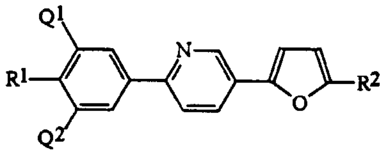

(1-32)

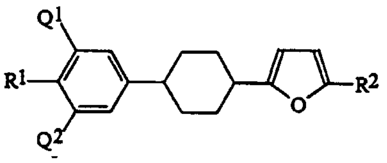

(1-33)

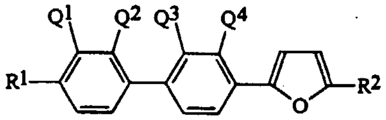

(1-34)

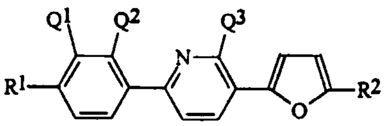

(1-35)

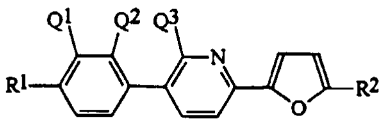

(1-36)

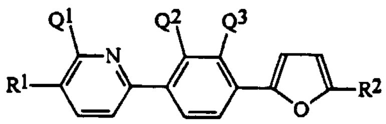

(1-37)

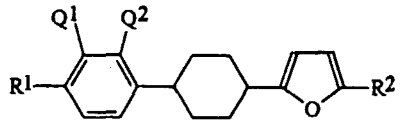

(1-38)

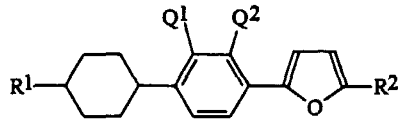

(1-39)

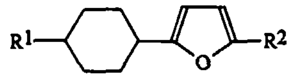

(1-40)

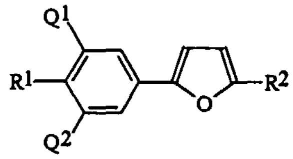

(1-41)

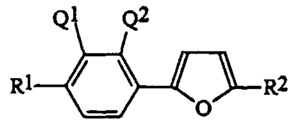

(1-42)

In the formulae (1-1) to (1-42) $Q^1$, $Q^2$, $Q^3$, $Q^4$, $Q^5$ and $Q^6$ are each independently a hydrogen atom or a halogen atom. $R^1$ in the formulae (1-1) to (1-42) is preferably a halogen atom or a group selected from -CN, $-CF_3$, $-OCF_3$, or an alkyl group, an alkoxy group, an alkoxyalkyl group, an alkenyl group, an alkenyloxy group, an alkenyloxyalkyl group or an alkyloxyalkenyl group having 1 to 12 carbon atoms, and particularly preferable examples thereof include methyl, ethyl, propyl, butyl, pentyl, hexyl, heptyl, octyl, methoxy, ethoxy, propoxy, butoxy, pentyloxy, hexyloxy, heptyloxy, octyloxy, methoxymethyl, ethoxymethyl, propoxymethyl, butoxymethyl, methoxyethyl, ethoxyethyl, propoxyethyl, methoxypropyl, ethoxypropyl, propoxypropyl, vinyl, 1-propenyl, 2-propenyl, 1-butenyl, 2-butenyl, 3-butenyl, 1-pentenyl, 2-pentenyl, 3-pentenyl, 4-pentenyl, 2-propenyloxy, 2-butenyloxy, 2-pentenyloxy, 4-pentenyloxy, methoxy-1-propenyl, methoxy-1-pentenyl and methoxy-3-pentenyl. $R^2$ in the formulae (1-1) to (1-42) is an alkyl group, an alkoxy group, an alkoxyalkyl group, an alkenyl group, an alkenyloxy group, an alkenyloxyalkyl group or an alkyloxyalkenyl group having 1 to 12 carbon atoms, and particularly preferable examples thereof include methyl, ethyl, propyl, butyl, pentyl, hexyl, heptyl, octyl, methoxy, ethoxy, propoxy, butoxy, pentyloxy, hexyloxy, heptyloxy, octyloxy, methoxymethyl, ethoxymethyl, propoxymethyl, butoxymethyl, methoxyethyl, ethoxyethyl, propoxyethyl, methoxypropyl, ethoxypropyl, propoxypropyl, vinyl, 1-propenyl, 2-propenyl, 1-butenyl, 2-butenyl, 3-butenyl, 1-pentenyl, 2-pentenyl, 3-pentenyl, 4-pentenyl, 2-propenyloxy, 2-butenyloxy, 2-pentenyloxy, 4-pentenyloxy, methoxy-1-propenyl, methoxy-1-pentenyl and methoxy-3-pentenyl.

A liquid crystal composition of the present invention contains 0.1 to 99.9% by weight, preferably 1 to 50% by weight, more preferably 3 to 20% by weight of one or more of the compounds (1), and this composition expresses excellent characteristics.

Another liquid crystal composition which can be provided by the present invention contains at least one of the compounds (1) as a first component and another compound optionally selected from the group consisting of compounds represented by the formulae (2) to (12) in compliance with the purpose of the liquid crystal composition.

Preferable examples of the compounds represented by the formulae (2) to (4) include the following compounds:

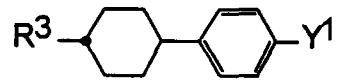
(2-1)

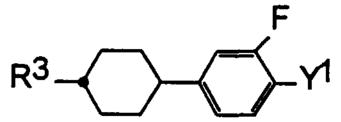
(2-2)

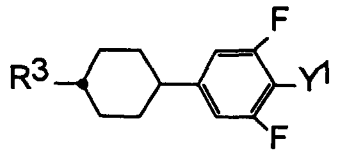
(2-3)

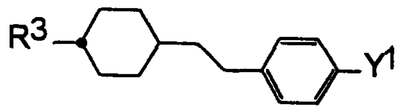
(2-4)

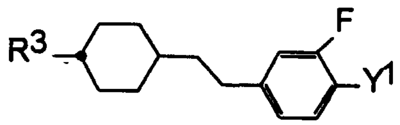
(2-5)

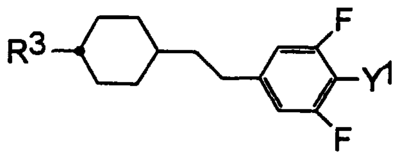
(2-6)

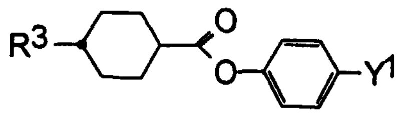
(2-7)

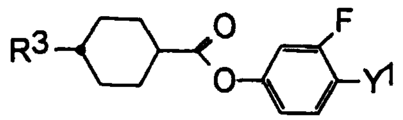
(2-8)

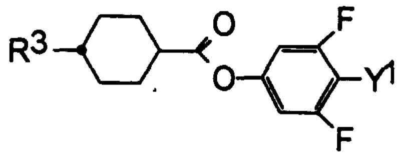
(2-9)

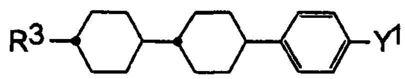

(3-1)

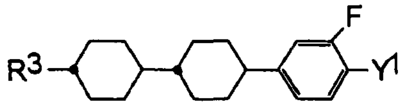

(3-2)

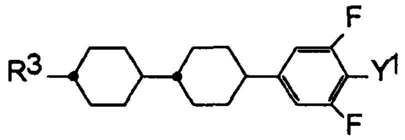

(3-3)

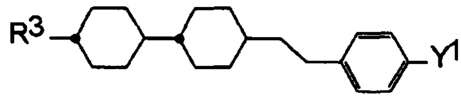

(3-4)

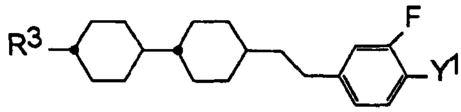

(3-5)

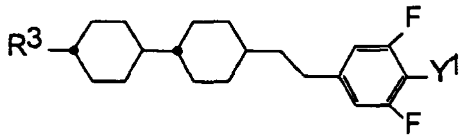

(3-6)

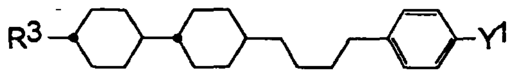

(3-7)

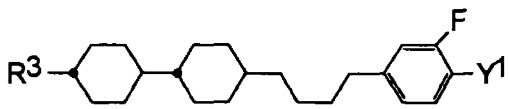

(3-8)

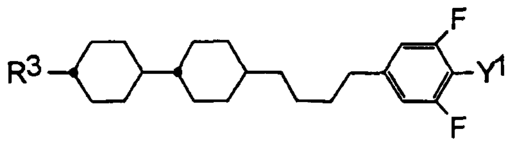

(3-9)

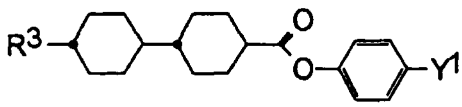

(3-10)

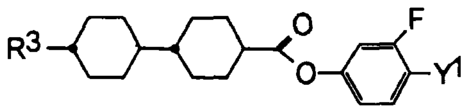

(3-11)

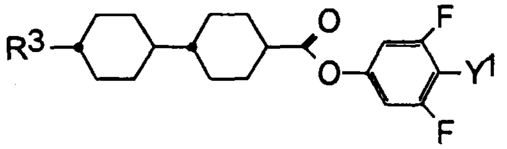

(3-12)

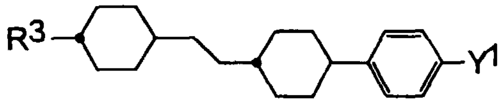

(3-13)

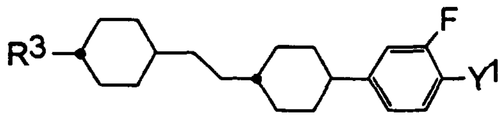

(3-14)

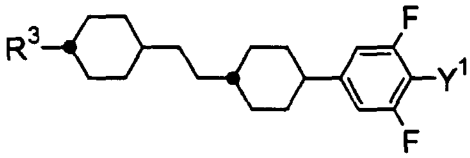

(3-15)

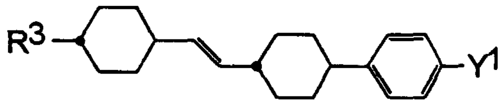

(3-16)

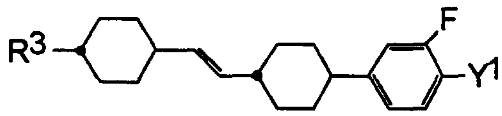

(3-17)

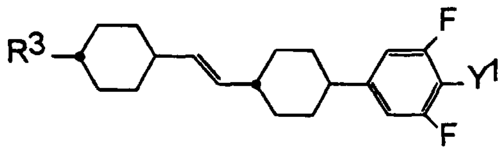

(3-18)

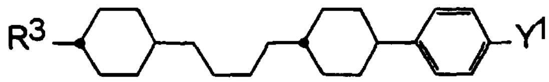

(3-19)

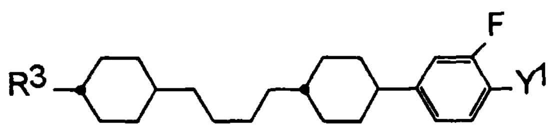

(3-20)

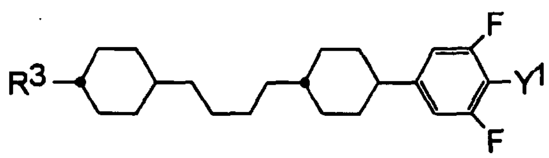

(3-21)

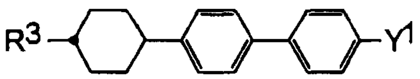
(3-22)

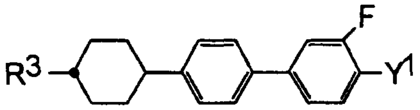
(3-23)

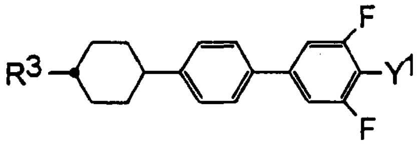
(3-24)

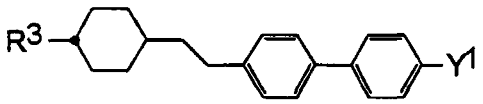
(3-25)

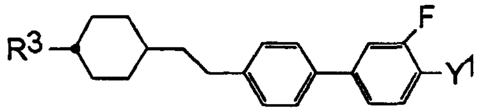
(3-26)

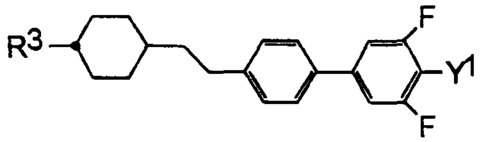
(3-27)

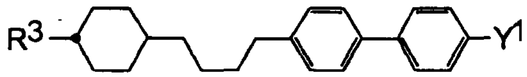
(3-28)

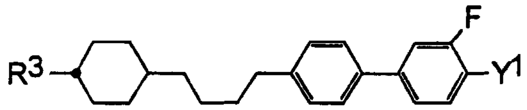
(3-29)

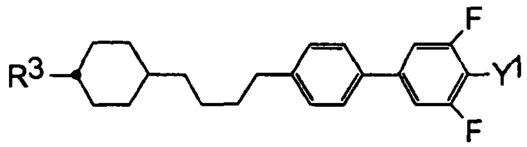
(3-30)

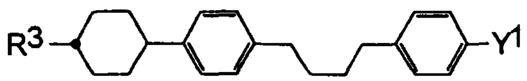
(3-31)

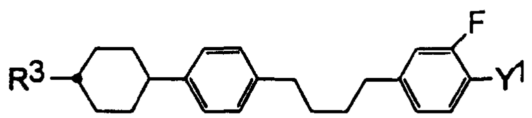
(3-32)

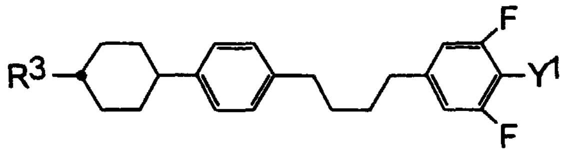

(3-33)

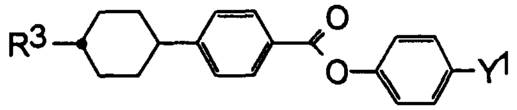

(3-34)

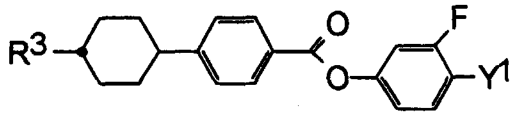

(3-35)

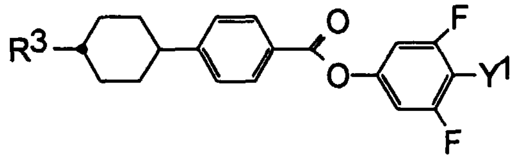

(3-36)

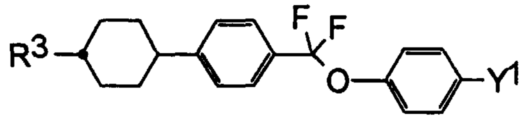

(3-37)

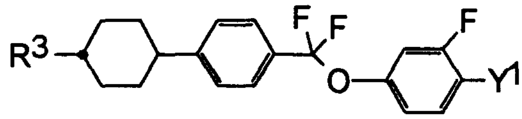

(3-38)

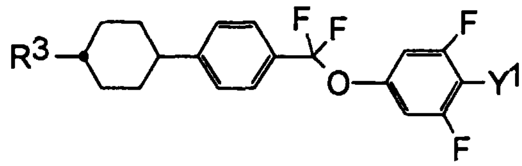

(3-39)

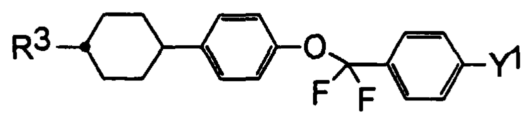

(3-40)

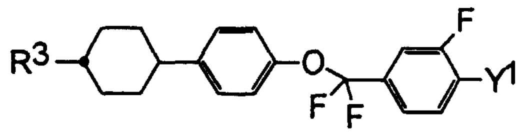

(3-41)

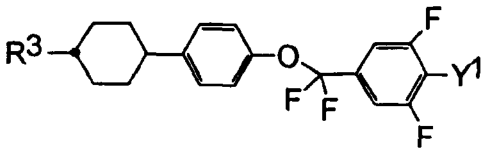

(3-42)

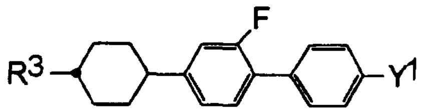

(3-43)

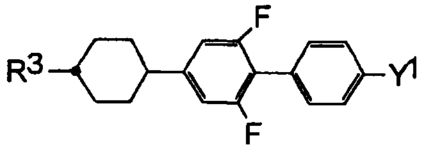

(3-44)

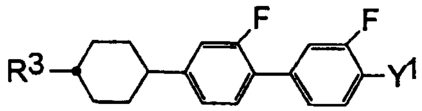

(3-45)

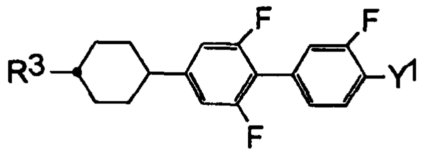

(3-46)

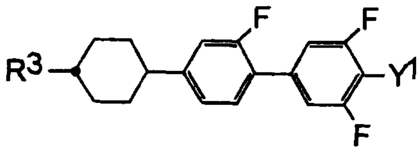

(3-47)

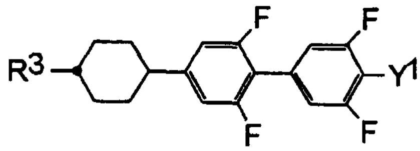

(3-48)

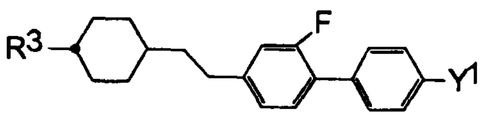

(3-49)

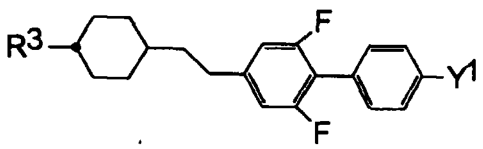

(3-50)

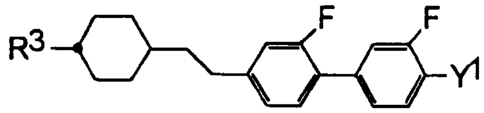

(3-51)

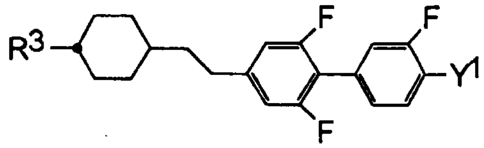

(3-52)

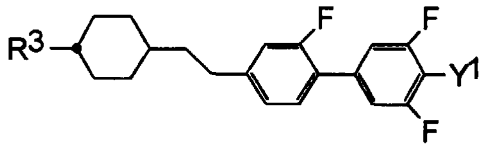

(3-53)

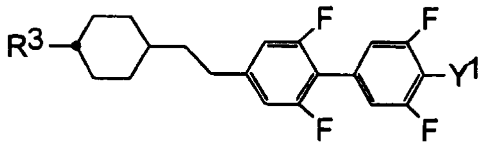

(3-54)

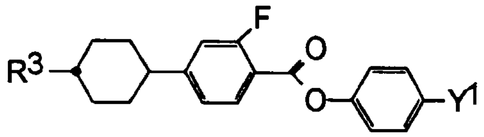

(3-55)

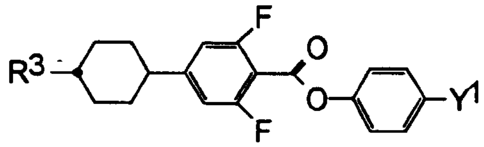

(3-56)

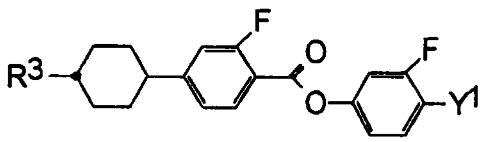

(3-57)

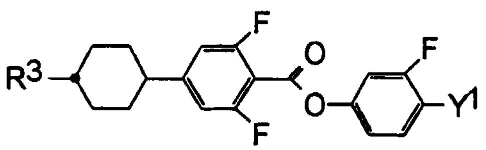

(3-58)

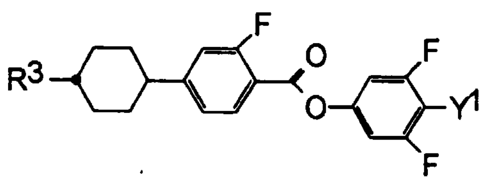

(3-59)

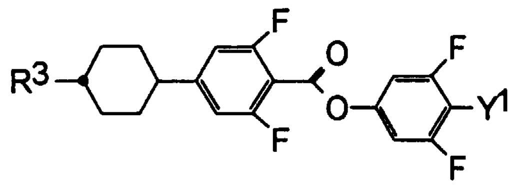

(3-60)

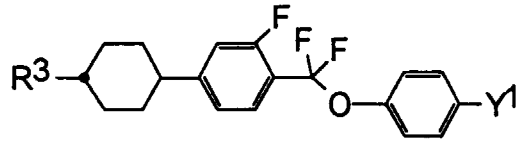

(3-61)

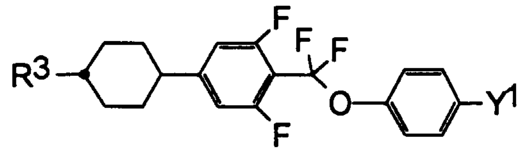

(3-62)

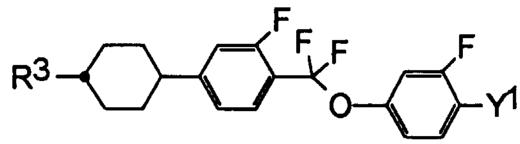

(3-63)

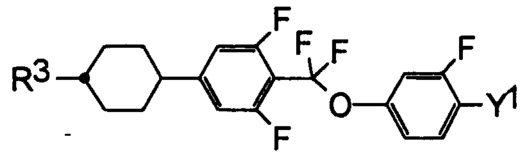

(3-64)

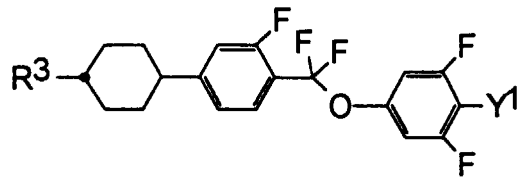

(3-65)

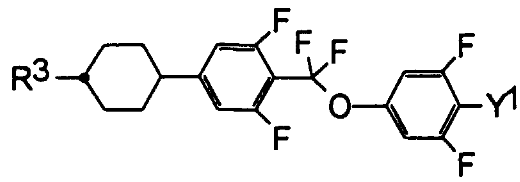

(3-66)

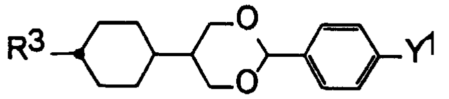

(3-67)

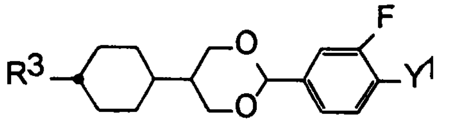

(3-68)

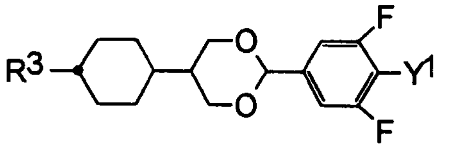

(3-69)

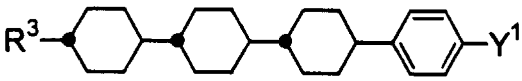

(4-1)

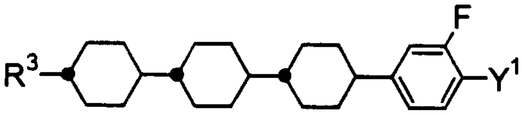

(4-2)

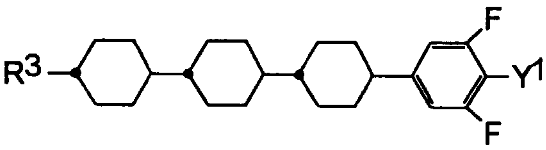

(4-3)

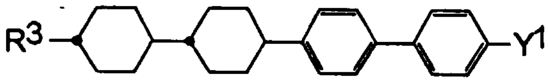

(4-4)

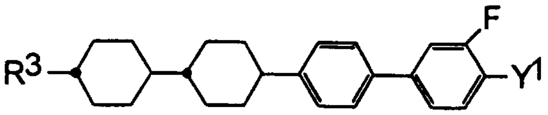

(4-5)

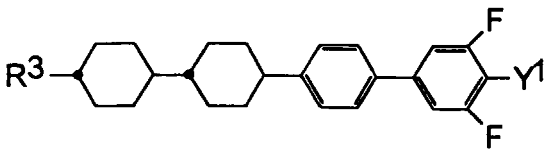

(4-6)

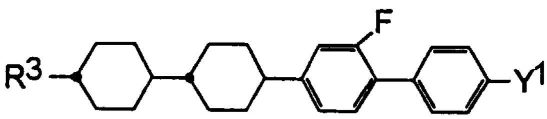

(4-7)

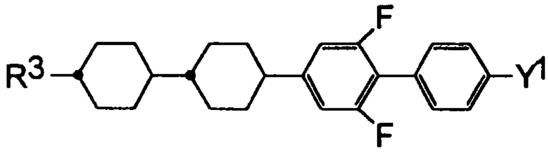

(4-8)

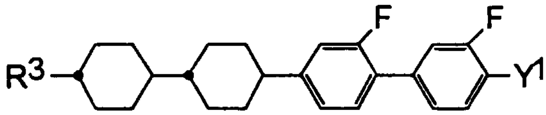

(4-9)

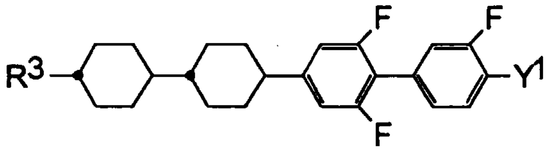

(4-10)

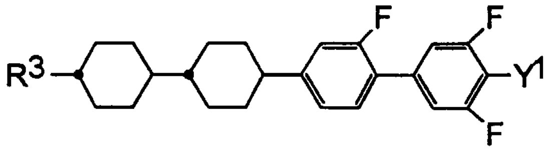

(4-11)

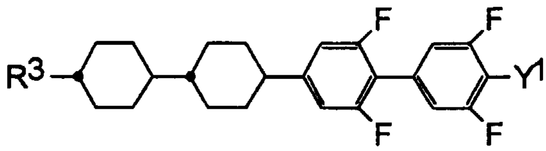

(4-12)

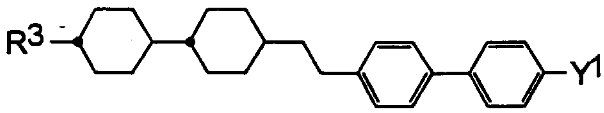

(4-13)

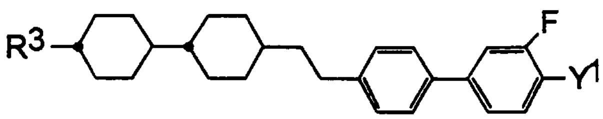

(4-14)

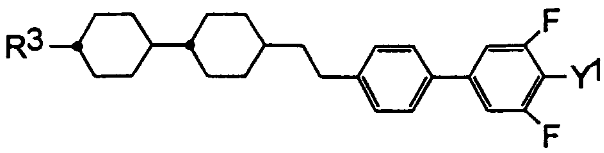

(4-15)

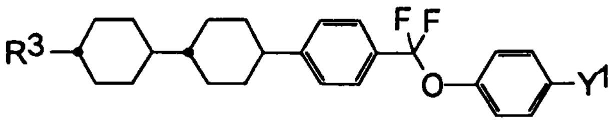

(4-16)

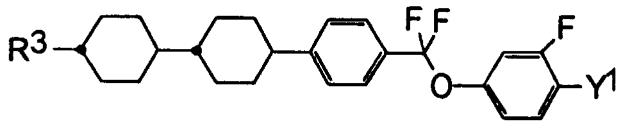

(4-17)

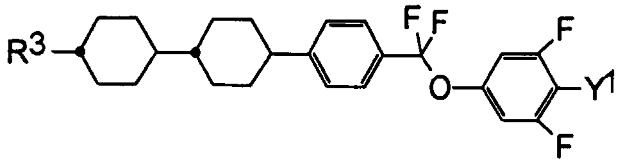

(4-18)

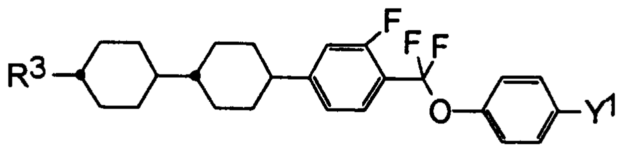

(4-19)

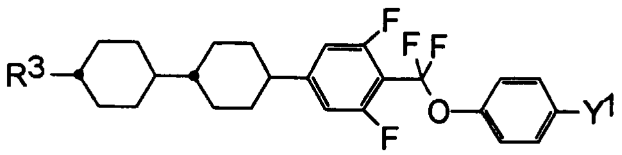

(4-20)

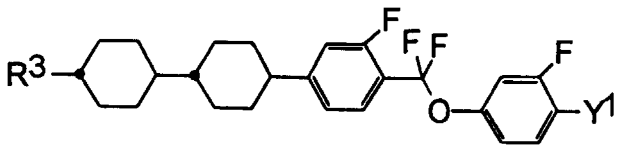

(4-21)

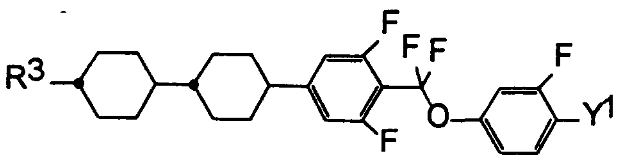

(4-22)

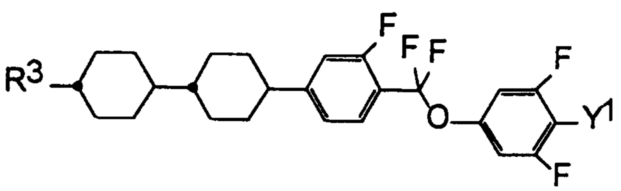

(4-23)

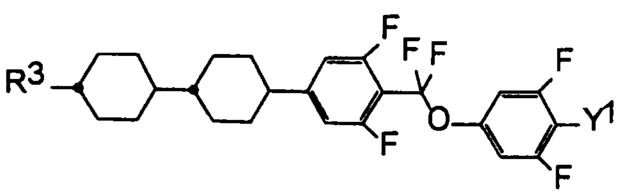

(4-24)

wherein $R^3$ and $Y^1$ are as defined above.

The compounds represented by the formulae (2) to (4) have positive dielectric anisotropy values, and they are essential to prepare a liquid crystal composition for TFT (AM-LCD) in which highly reliable characteristics such as an excellent thermal and chemical stability as well as high voltage holding ratio (or a high specific resistance) are

required.

In the case of preparing the liquid crystal composition for TFT, the amount of any compound of the formulae (2) to (4) used is in the range of 0.1 to 99% by weight, preferably 10 to 97% by weight, more preferably 40 to 95% by weight with respect to the total weight of the liquid crystal composition. In preparing the liquid crystal composition for TFT, any of the compounds of the formulae (7) to (9) may be used for the adjustment of the viscosity. Also in preparing the liquid crystal composition for a STN display system or a TN display system, any of the compounds of the formulae (2) to (4) may be used. In this case, the amount of any compound of the formulae (2) to (4) is preferably 50% by weight or less, because the compounds have a less effect of decreasing the threshold voltage of the liquid crystal composition compared with the compounds of the formulae (5) and (6).

Preferable examples of the compounds represented by the formulae (5) to (6) include the following compounds:

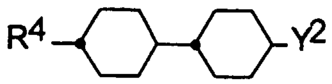

(5-1)

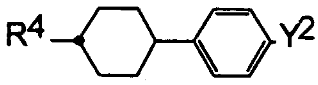

(5-2)

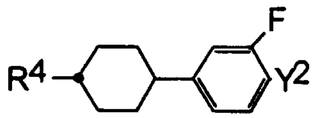

(5-3)

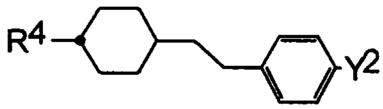

(5-4)

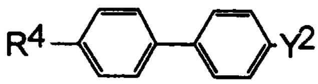

(5-5)

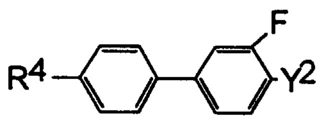

(5-6)

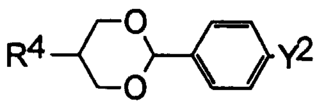

(5-7)

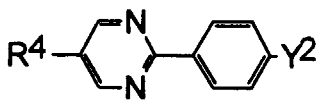

(5-8)

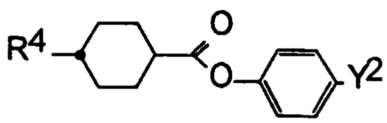

(5-9)

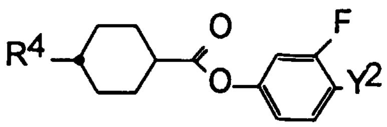

(5-10)

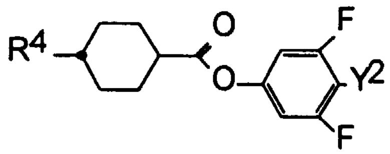

(5-11)

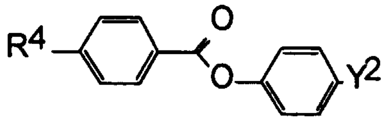

(5-12)

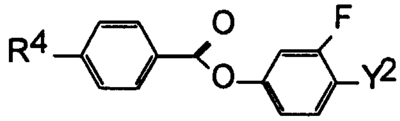

(5-13)

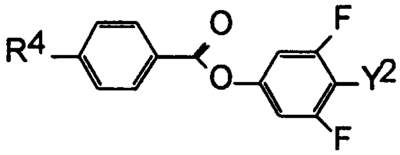

(5-14)

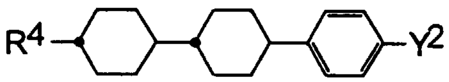

(5-15)

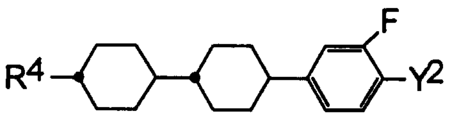

(5-16)

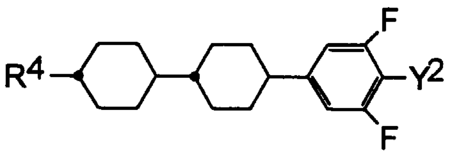

(5-17)

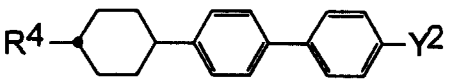

(5-18)

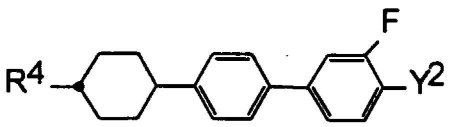

(5-19)

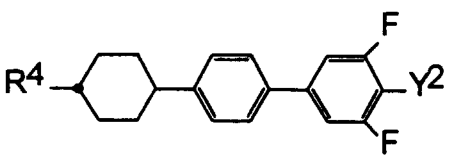

(5-20)

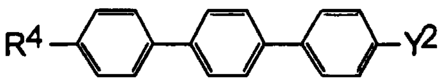

(5-21)

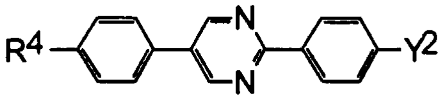

(5-22)

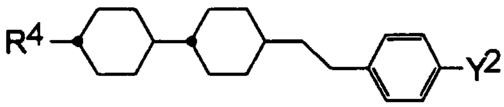

(5-23)

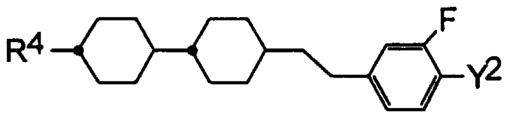

(5-24)

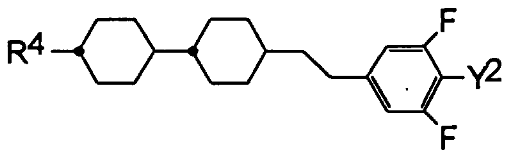

(5-25)

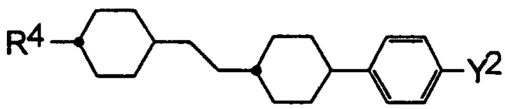

(5-26)

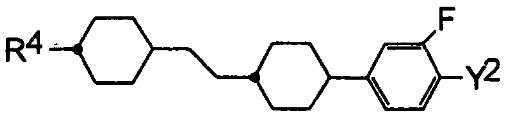

(5-27)

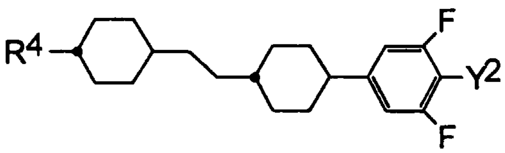

(5-28)

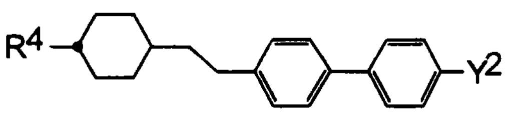

(5-29)

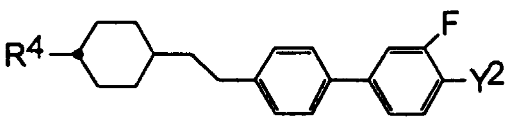

(5-30)

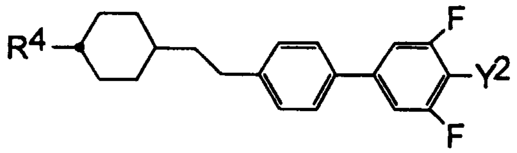
(5-31)

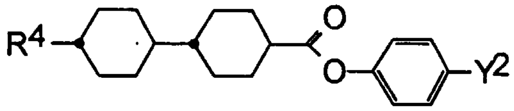
(5-32)

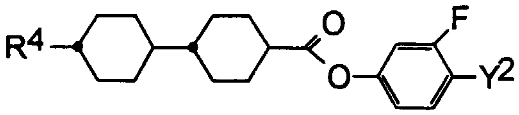
(5-33)

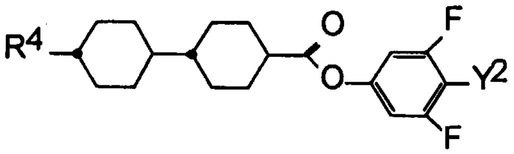
(5-34)

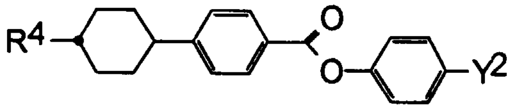
(5-35)

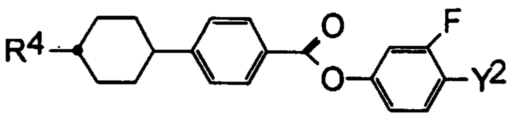
(5-36)

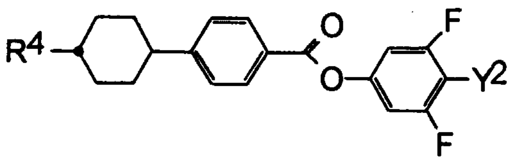
(5-37)

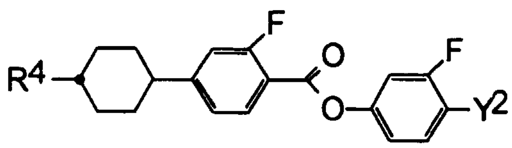
(5-38)

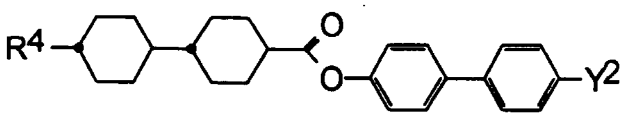
(5-39)

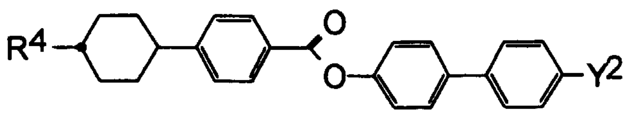

(5-40)

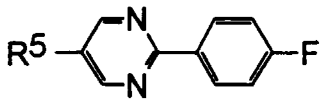

(6-1)

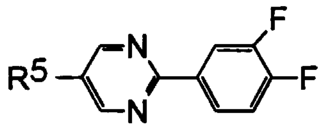

(6-2)

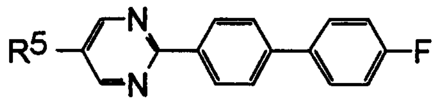

(6-3)

wherein $R^4$, $R^5$ and $Y^2$ are as defined above.

The compounds of the formulae (5) and (6) have large positive dielectric anisotropy values, and they can be used for the purpose of decreasing the threshold voltage. Furthermore, they can also be used for the purpose of enlargement of the nematic temperature range, the adjustment of the optical anisotropy value or the like. Alternatively they can also be used in order to improve the steepness of the threshold voltage of the liquid crystal composition for a STN display system or a TN display system.

The compounds of the formulae (5) and (6) are essential for the preparation of a liquid crystal composition for a STN display system or a TN display system.

If the amount of the compounds of the formulae (5) and (6) is increased, the threshold voltage of the liquid crystal composition decreases and the viscosity increases. Therefore, it is advantageous to use the compound of the formula (5) or (6) in the largest possible amount so as to meet the required viscosity of the liquid crystal composition, because such a constitution permits the drive of the display at a low voltage.

In the case of preparing the liquid crystal composition for a STN display system or a TN display system, the amount of the compounds of the formulae (5) and (6) used is in the range of 0.1 to 99.0% by weight, preferably 10 to 97% by weight, more preferably 40 to 95% by weight with respect to the total weight of the liquid crystal composition.

Preferable examples of the compounds represented by the formulae (7) to (9) include the following compounds:

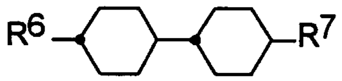

(7-1)

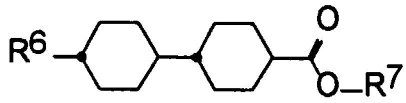

(7-2)

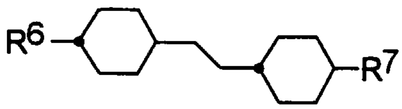

(7-3)

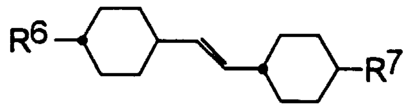

(7-4)

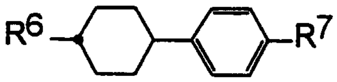

(7-5)

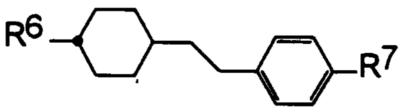

(7-6)

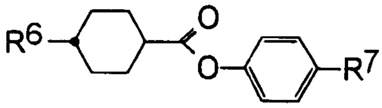

(7-7)

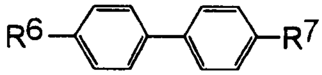

(7-8)

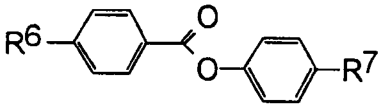

(7-9)

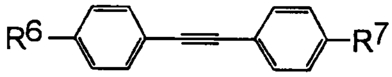

(7-10)

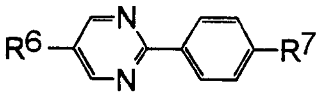

(7-11)

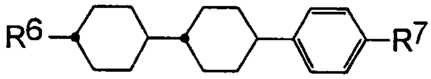

(8-1)

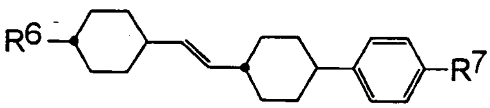

(8-2)

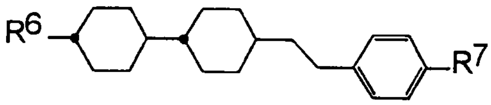

(8-3)

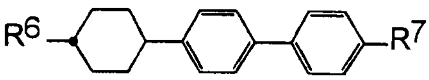

(8-4)

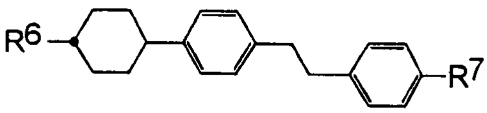

(8-5)

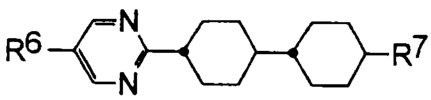

(8-6)

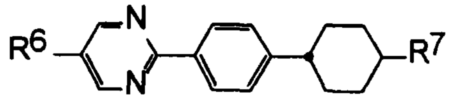

(8-7)

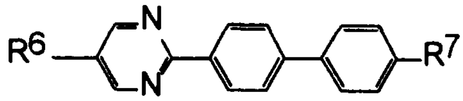

(8-8)

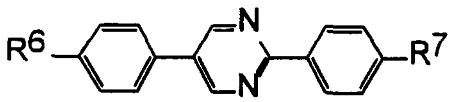

(8-9)

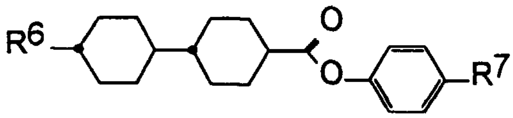

(8-10)

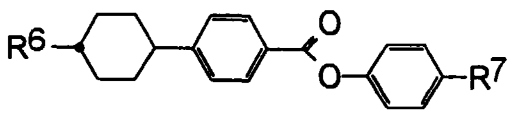

(8-11)

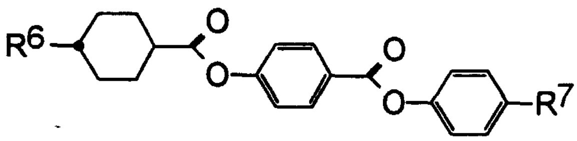

(8-12)

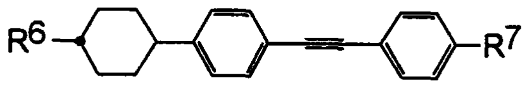

(8-13)

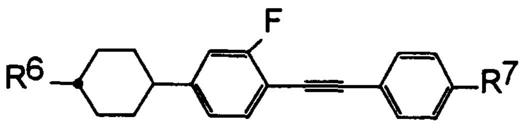

(8-14)

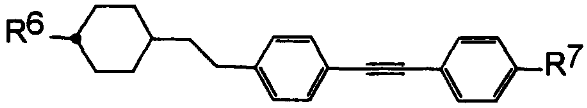

(8-15)

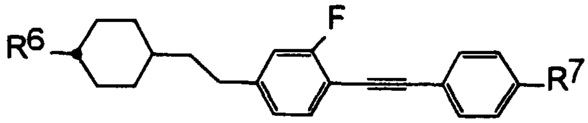

(8-16)

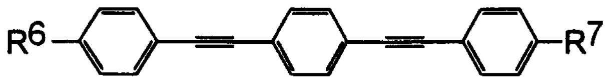

(8-17)

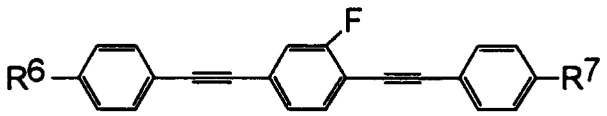

(8-18)

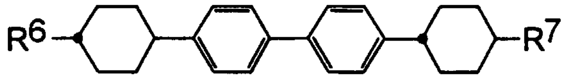

(9-1)

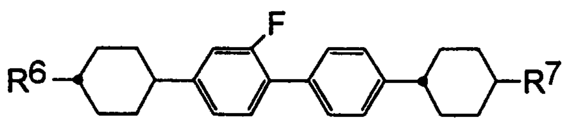

(9-2)

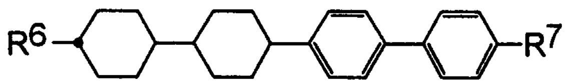

(9-3)

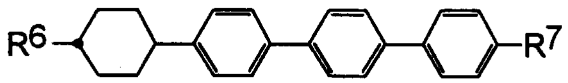

(9-4)

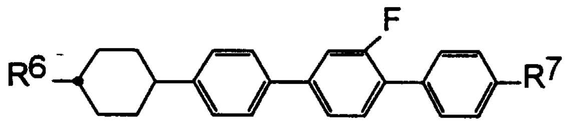

(9-5)

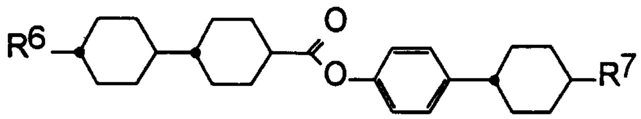

(9-6)

wherein $R^6$ and $R^7$ are as defined above.

The compounds represented by the formulae (7) to (9) have small absolute values of the dielectric anisotropy, and so they are nearly neutral. The compounds of the formula (7) are mainly used for the purpose of adjusting the viscosity or the optical anisotropy value. Furthermore, the compounds of the formulae (8) and (9) are used for the purpose of enlargement of the nematic range such as the rise of the clearing point or the like, or for the purpose of the adjustment of the optical anisotropy value.

When the amount of any compound of the formulae (7) to (9) is increased, the threshold voltage of the liquid crystal composition increases and the viscosity decreases. Therefore, it is desirable to use the compound in the largest possible amount so as to meet the required threshold voltage. In the case of preparing a liquid crystal composition for a TFT display system, the amount of the compounds of the formulae (7) to (9) used is in the range of 40% by weight or less, preferably 35% by weight or less with respect to the total weight of the liquid crystal composition. In the case of preparing the liquid crystal composition for a STN display system or a TN display system, the amount of the compound of the formulae (7) to (9) used is 70% by weight or less, preferably 60% by weight or less.

Preferable examples of the compounds represented by the formulae (10) to (12) include the following compounds:

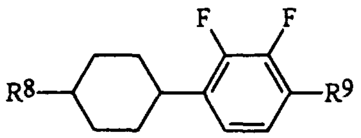

(10-1)

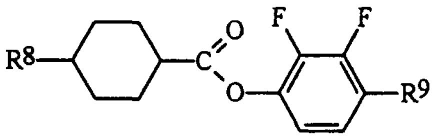

(10-2)

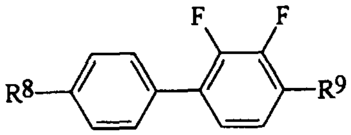

(10-3)

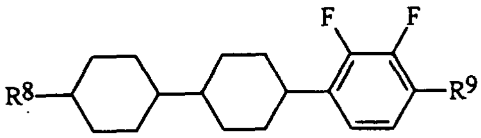

(11-1)

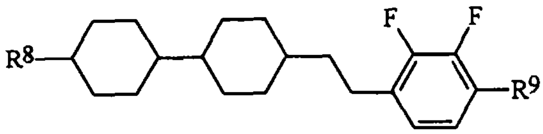

(11-2)

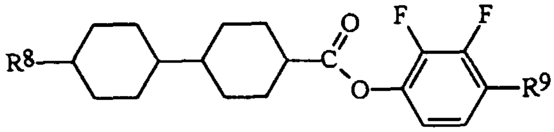

(11-3)

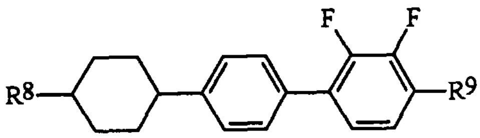

(11-4)

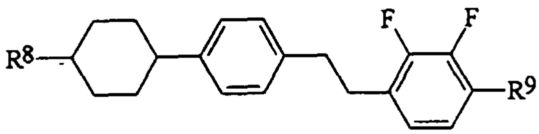

(11-5)

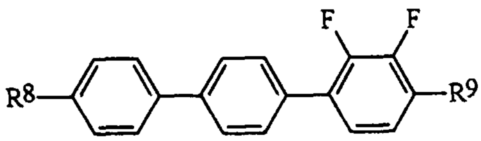

(12-1)

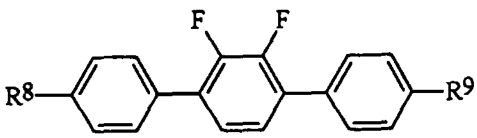

(12-2)

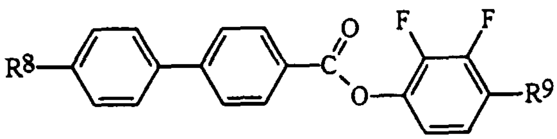

(12-3)

wherein $R^8$ and $R^9$ are as defined above.

The compounds represented by the formulae (10) to (12) have negative dielectric anisotropy values. The compounds represented by the formula (10) mainly are used in order to adjust the threshold voltage, the viscosity

and/or the optical anisotropy value of a liquid crystal composition. The compounds represented by the formula (11) are used in order to extend the nematic range of a liquid crystal composition and to increase the clearing temperature or to control the value of the optical anisotropy. The compounds represented by the formula (12) are used for the purpose of extending the nematic range, decreasing the threshold voltage and/or increasing the value of the optical anisotropy.

The compounds represented by the formulae (10) to (12) are usually used for liquid crystal compositions with negative dielectric anisotropy value (N-type). When the content of the compounds represented by the formulae (10) to (12) is increased, the threshold voltage of the liquid crystal composition decreases but the viscosity of the liquid crystal composition increases. Therefore, it is advantageous to use the compounds of the formulae (10) to (12) in the smallest possible amount so as to meet the required threshold voltage of the liquid crystal composition. However, since the absolute value of the dielectric anisotropy of these compounds is smaller than 5, an amount of 40% by weight of the compounds of the formulae (10) to (12) may be necessary to drive the display system at low voltage. With respect to this, the amount of the compounds of the formulae (10) to (12) in a N-type liquid crystal composition for a TFT display system is preferably higher than 40% by weight, more preferably 50 to 95% by weight with respect to the total weight of the liquid crystal composition.

There is also the case that the compounds represented by the formulae (10) to (12) are used in a liquid crystal composition with positive dielectric anisotropy value (P-type), in order to adjust the elastic constants of the liquid crystal composition and/or in order to restrict the transmission-voltage curve (V-T-curve). In this case, the amount of the compounds of the formulae (10) to (12) used is preferably 30% by weight or less with respect to the total weight of the liquid crystal composition.

Furthermore, except a special case of a liquid crystal composition for an OCB (optically compensated birefringence) mode or the like, an optically active compound may be added to the liquid crystal composition usually for the purpose of forming a helical structure of the liquid crystal composition, adjusting a necessary twist angle and preventing reverse twist. For such purposes any of the known optically active compounds may be used, but the following optically active compounds are preferable:

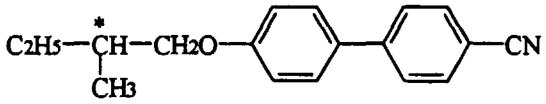

(C15)

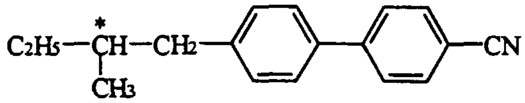

(CB15)

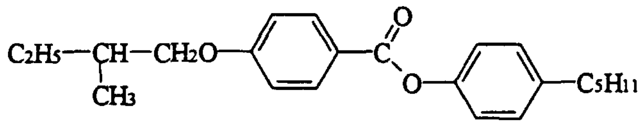

(CM21)

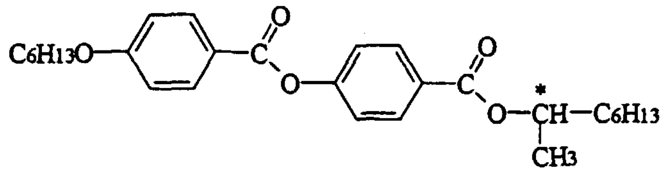

(CM33)

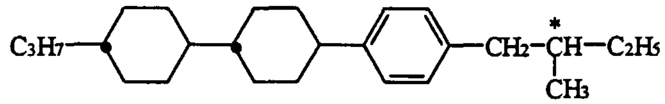

(CM43-L)

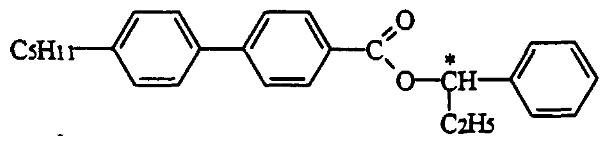

(CM45)

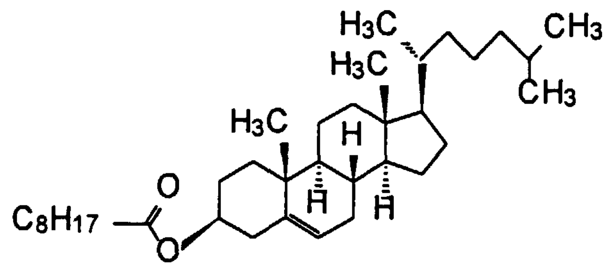

(CN)

Any of these optically active compounds is usually added to the liquid crystal composition of the present invention to adjust the pitch length of the twist. In the cases that the liquid crystal composition is used for TFT or TN display systems, the pitch length of the twist is preferably adjusted so as to be in the range from 40 to 200 μm. In the case that the liquid crystal composition is used for STN display systems, the pitch length of the twist is preferably adjusted so as to be in the range of 6 to 20 μm. Moreover, in the case of a bistable TN mode, the pitch of the twist is preferably adjusted so as to be in the range of 1.5 to 4 μm. In addition, for the purpose of adjusting the dependency of the pitch length on temperature, two or more different optically active compounds may be added.

The liquid crystal composition of the present invention can be prepared in a conventional manner. In general, a method which comprises mutually dissolving the above various components at high temperature can be employed.

Furthermore, the liquid crystal composition of the present invention can be used as a liquid crystal composition for a guest-host (GH) mode by adding a dichromatic dye such as a merocyanine dye, a styryl dye, an azo dye, an

azomethine dye, an azoxy dye, a quinophthalone dye, an anthraquinone dye or a tetrazine dye thereto. Moreover, the liquid crystal composition of the present invention can also be used as a liquid crystal composition for NCAP in which microcapsules containing the nematic liquid crystal are used, and as a liquid crystal composition for a polymer dispersed type liquid crystal display device (PDLCD) typified by a polymer network liquid crystal display device (PNLCD) in which a three-dimensional network polymer is formed in the liquid crystal.

In addition, the liquid crystal composition of the present invention can also be used as a liquid crystal composition for a double refraction control (ECB) mode and a dynamic scattering mode (DS) display system.

The compounds of the present invention represented by the formula (1) can be manufactured by employing known procedures in the field of organic synthesis, wherein suitable known reactions may be selected and combined and suitable reaction conditions may be used.

In the following description, Hal is a symbol for a halogen atom and M is a symbol for metal substituent which can be suitably selected from Li, ZnHal or MgHal. THF is a symbol for tetrahydrofuran.

Monosubstitution of furan (13) can be effected by metallation with sec.-buthyllithium in THF as a solvent according to the following reaction. Transmetallation can be effected by reaction with MHal, which is preferably $MgCl_2$ or $ZnCl_2$.

Equation 1

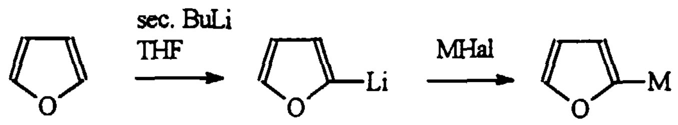


(13) (14)

Starting from compound (14), the moiety $-(X^4-A^4)_q-(X^5-A^5)_r-R^2$ of the formula (1) can be introduced, for example, by the following reactions:

Compound (14) is reacted with alkyl halogenides Hal-$R^{10}$ to give substituted furans (15).

Equation 2

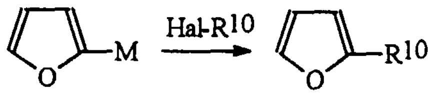


(14) (15)

where $R^{10}$ preferably represents an alkyl chain, an alkenyl chain, an alkoxyalkyl chain, an alkenyloxyalkyl chain or the moiety $-(CH_2-CH_2-A^4)_q-(X^5-A^5)_r-R^2$ wherein the symbols are as defined above and q is 1 or 2.

Reaction of compound (14) with ketones (16) gives the tertiary alcohol compounds (17) which can be dehydrated by acidic treatment. The substituted furans (19) can be obtained by catalytic hydrogenation of compound (18). In case that $R^{11}$ includes unsaturated groups, a suitable catalyst for the catalytic hydrogenation has to be selected.

Equation 3

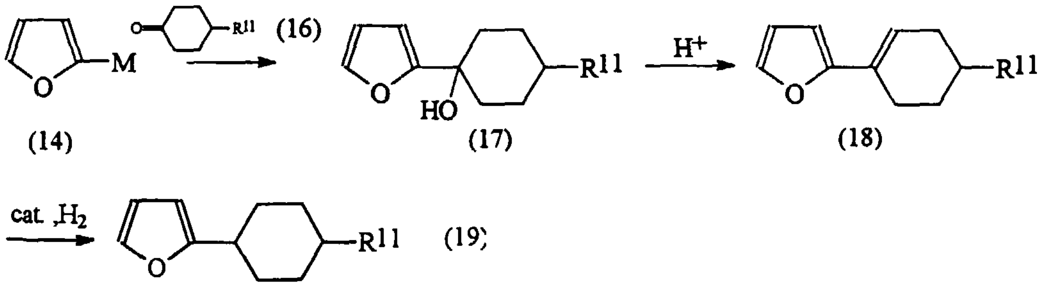


(14) (17) (18) (19)

where $R^{11}$ represents the moiety $-(X^5-A^5)_r-R^2$ wherein the symbols are as defined above.

Transmetallation of compound (14) and hydrolysis gives the boric acid (21), which can be subjected to cross-coupling reaction with arylhalides (22) to give the 2-arylfurans (23).

Equation 4

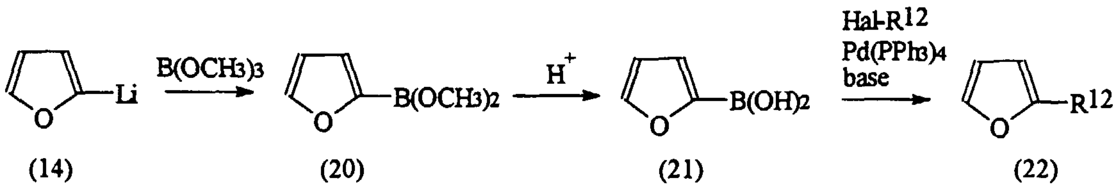


(14) (20) (21) (22)

where $R^{12}$ represents the moiety $-(A^4)_q-(X^5-A^5)_r-R^2$ wherein $X^5$, $A^5$, r and $R^2$ are as defined above and $A^4$ can be selected from the group consisting of 1,4-phenylene in which one or more hydrogen atom(s) may optionally be substituted with a halogen atom or a $CF_3$ group, pyrimidine-2,5-diyl, pyridine-2,5-diyl in which one hydrogen atom may be substituted with a halogen atom, thiadiazole-2,5-diyl and thiophene-2,5-diyl, and q is 1 or 2.

The boric acid (21) can be oxidized to the hydroxyfuran (23) by treatment with hydrogen peroxide. Etherification with suitable halides gives the ether compounds (24) and (25).

Equation 5

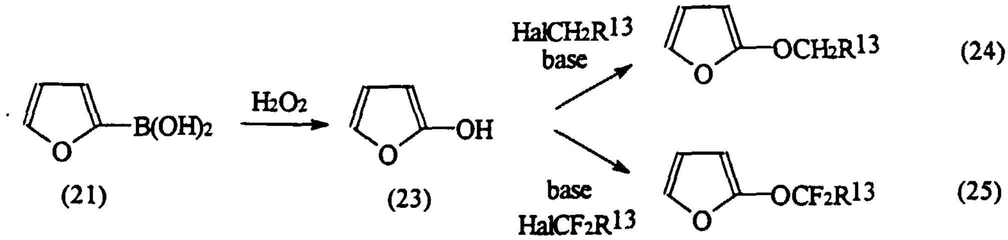


(21) (23) (24) (25)

where $R^{13}$ represents an alkyl chain, an alkenyl chain, a fluorinated or semifluorinated alkyl chain, an alkoxyalkyl chain, an alkenyloxyalkyl chain or the moiety $-(A^4)_q-(X^5-A^5)_r-R^2$ where $A^4$, $X^5$, $A^5$, r and $R^2$ are as defined above and q is 1.

Starting from compound (14), furan-2-carboxylic acid (26) can be prepared by treatment with carbon dioxide. Esterification gives the ester (27) which can be transferred into the thioester (28). Fluorination gives compound (29).

Equation 6

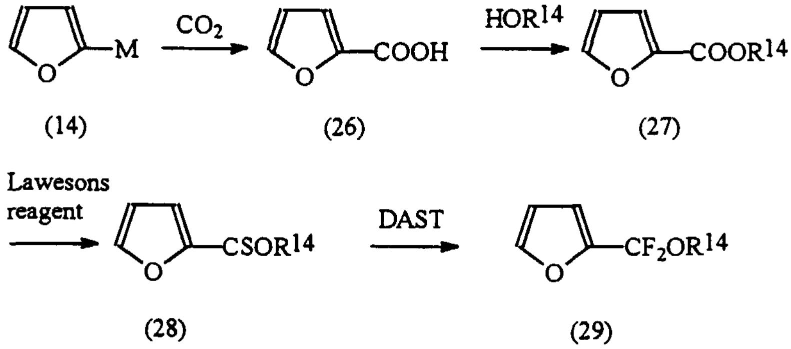


where $R^{14}$ represents an alkyl chain, an alkenyl chain, a fluorinated or semifluorinated alkyl chain, an alkoxyalkyl chain, an alkenyloxyalkyl chain or the moiety $-(A^4)_q-(X^5-A^5)_r-R^2$ where $A^4$, $X^5$, $A^5$, r and $R^2$ are as defined above and q is 1.

The Lithium atom at compound (14) can be replaced by iodine to give iodide (30). The iodide (30) undergoes palladium catalyzed coupling reactions with olefines to give compound (31) and with acetylenes to give compound (32).

Equation 7

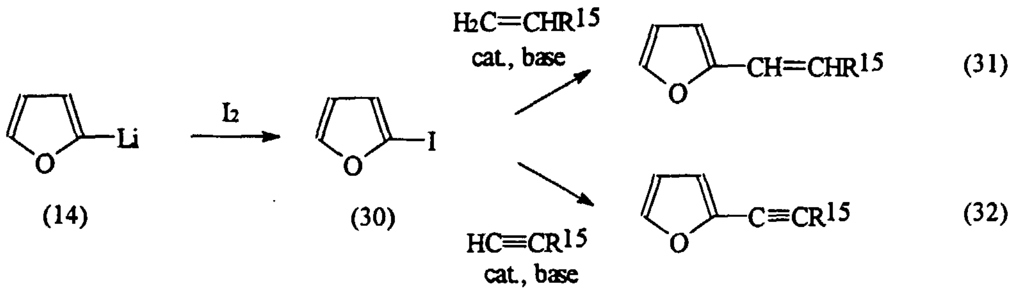


$R^{15}$ represents an alkyl chain, an alkoxyalkyl chain, an fluorinated or semifluorinated alkyl chain or the moiety $-(A^4)_q-(X^5-A^5)_r-R^2$ wherein the symbols are as defined above and q is 1. If $R^2$ includes a terminal olefin, the reaction condition, e.g., catalyst and base have to be selected suitably.

Another suitable starting material for the synthesis of monosubstituted furans is furfural (33). Alkenyl chains can be introduced by a series of Wittig-reactions. For example, the synthesis of 2-allylfuran (36) is given below.

Equation 8

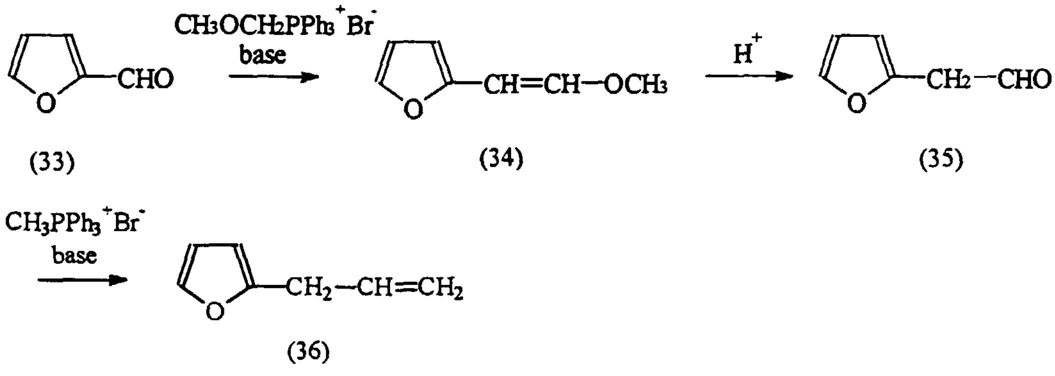


(33) (34) (35) (36)

Reduction of furfural (33) leads to furylmethanol (37), which can be transferred into the corresponding bromides (38). By etherification of bromides (38), various 2-oxymethyl-substituted furans (39) can be synthesized.

Equation 9

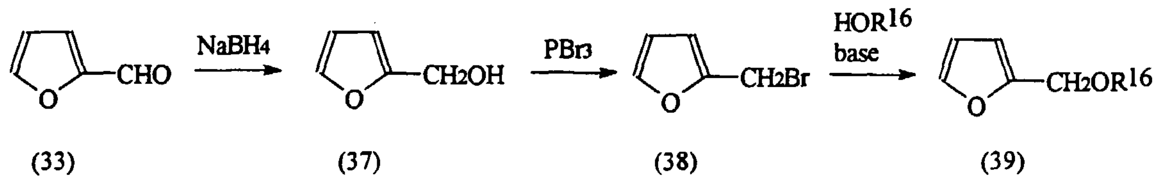


(33) (37) (38) (39)

wherein $R^{16}$ represents an alkyl chain, an alkenyl chain or the moiety $-(A^4)_q-(X^5-A^5)_r-R^2$ where $A^4$, $X^5$, $A^5$, r and $R^2$ are as defined above and q is 1.

Condensation of furfural (33) with 2-substituted propane-1,3-diol (40) under acidic catalysis leads to 2-(1,3-dioxane-2-yl)-furans (41).

Equation 10

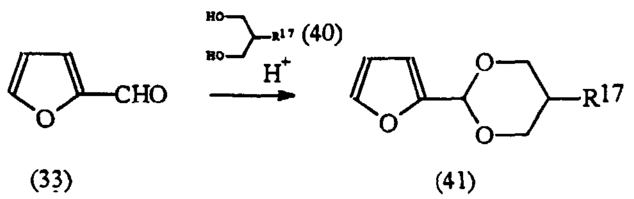


(33) (41)

wherein $R^{17}$ represents an alkyl chain or the moiety - $(X^5-A^5)_r-R^2$, where the symbols are as defined above. In case that $A^5$ is 1,3-dioxane-2,5-diyl, a mild catalyst has to be selected as proton source.

The compounds as synthesized above can generally be described by the formula (42) wherein the symbols are as defined above. Metallation of compound (42) can be effected with sec. buthyllithium in THF.

Equation 11

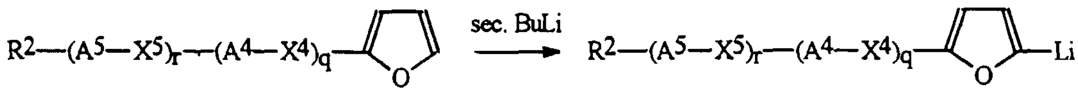


(42) (43)

In general, compound (43) undergoes the same reactions as described above, such as transmetallation, reaction with alkyl halides, ketones, carbon dioxide, trimethylborate and iodine, for example. Thus the compounds of the formula (1) can be synthesized employing the procedures as described above.

<u>Examples</u>

In the following description, Cr is a crystalline phase, SmA is a smectic phase of type A, SmB is a smectic phase of type B and so on, N is a nematic phase and Is is an isotropic liquid phase. cyc represents trans-1,4-cyclohexylene, phe represents 1,4-phenylene, 2F3Fphe represents 2,3-difluorophenylene, 3F5Fphe represents 3,5-difluorophenylene, 2Fphe represents 2-fluorophenylene, 3Fphe represents 3-fluorophenylene, 2pyr represents pyridine-2,5-diyl, 3pyr represents pyridine-3,6-diyl, 3F2pyr represents 3-fluoro-pyridine-2,5-diyl, 2F3pyr represents 2-fluoro-pyridine-3,6-diyl and py represents pyrimidine-2,5-diyl.

Example 1

Synthesis of 5-(4'-trans-pentyl-trans-bicyclohexyl-4-yl)-2-methylfuran

No. 1

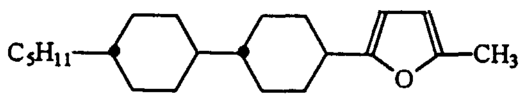

Step 1.1

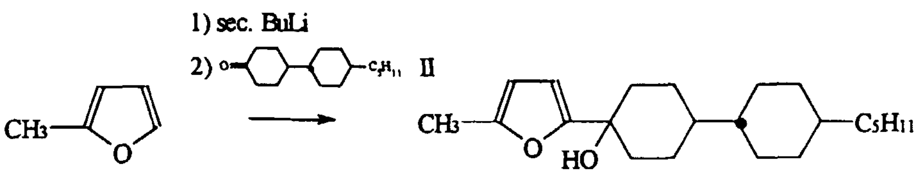


I III

To a solution of compound I (0.5 mol) in 500 ml of THF, 0.5 mol of sec. BuLi is added at -60°C. After stirring for 1 hour at the same temperature, a solution of compound II (0.45 mol) in 300 ml of THF is added at a temperature between -60 and -50°C. The reaction mixture is allowed to warm up to room temperature and 1 l of water is added. The

organic layer is separated and the aqueous layer is extracted with acetic ester. The solvent of the combined organic layers is distilled off under reduced pressure. The crude product is recrystallized from ethanol.

Step 1.2

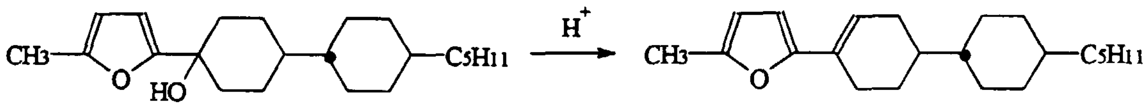


III IV

Cr 40.2 SmB 53.9 SmA 60.0 N 61.5 Is

To a solution of compound III (0.4 mol) in 500 ml of acetic ester, 50 ml of 6 N aqueous solution of HCl is given. The mixture is stirred at room temperature overnight. The organic layer is separated and the solvent is evaporated. Recrystallization from ethanol gives compound IV.

Step 1.3

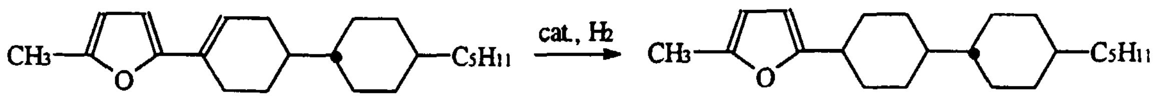


IV V

0.38 mol of compound IV is dissolved in 800 ml of ethanol. 10 g of Raney-Nickel are added and hydrogenation is carried out at room temperature and a pressure of 760 mmHg. The catalyst is filtered off and the solvent is evaporated.

Step 1.4

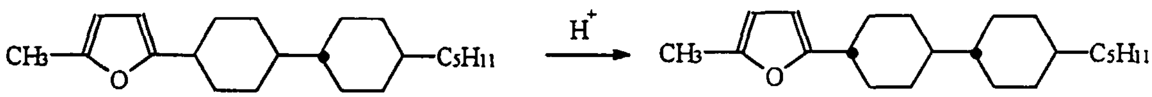


V VI

0.1 mol of compound V is dissolved in 300 ml of toluene and 2 g of 4-toluenesulphonic acid are added. The solution is refluxed for 6 hours. After cooling the solution is washed with water, the organic layer is dried over $MgSO_4$ and the solvent is distilled off. The crude product is subjected to a column chromatography with n-heptane as eluent and recrystallized from ethanol. Cr 15.3 SmB 87.1 Is

The following analogous compounds,

Compounds 2 to 56,

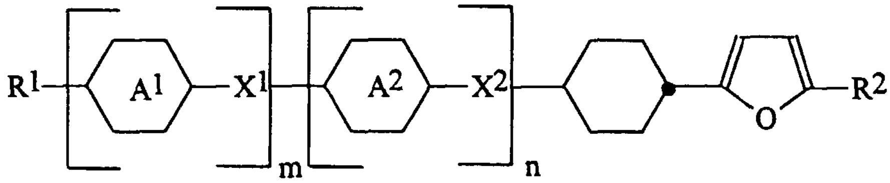

are synthesized.

Table 1

| No. | $R^1$ | $A^1$ | $X^1$ | m | $A^2$ | $X^2$ | n | 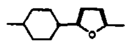 | $R^2$ |
|---|---|---|---|---|---|---|---|---|---|
| 2 | $C_5H_{11}$ | | | 0 | | | 0 | | $CH_3$ |
| 3 | $C_3H_7$ | | | 0 | | | 0 | | $C_2H_5$ |
| 4 | $C_4H_9$ | | | 0 | | | 0 | | $C_7H_{15}$ |
| 5 | $C_2H_5$ | | | 0 | | | 0 | | $OCH_3$ |
| 6 | $C_5H_{11}$ | | | 0 | | | 0 | | $OC_5H_{11}$ |
| 7 | $C_3H_7$ | | | 0 | cyc | - | 1 | | $CH_3$ |
| 8 | $C_7H_{15}$ | | | 0 | cyc | - | 1 | | $C_3H_7$ |
| 9 | $CH_3$ | | | 0 | cyc | - | 1 | | $C_4H_9$ |
| 10 | $C_2H_5$ | | | 0 | cyc | - | 1 | | $C_6H_{13}$ |
| 11 | $C_3H_7$ | | | 0 | cyc | - | 1 | | $OCH_3$ |
| 12 | $C_5H_{11}$ | | | 0 | cyc | - | 1 | | $OC_3H_7$ |
| 13 | $C_4H_9$ | | | 0 | cyc | $-(CH_2)_2-$ | 1 | | $C_3H_7$ |
| 14 | $CH_3$ | | | 0 | cyc | $-(CH_2)_2-$ | 1 | | $C_7H_{15}$ |
| 15 | $C_9H_{19}$ | | | 0 | cyc | $-(CH_2)_2-$ | 1 | | $OCH_3$ |
| 16 | $C_5H_{11}$ | | | 0 | cyc | $-(CH_2)_2-$ | 1 | | $OC_2H_5$ |
| 17 | $C_3H_7$ | | | 0 | cyc | $-(CH_2)_4-$ | 1 | | $CH_3$ |
| 18 | $C_6H_{13}$ | | | 0 | cyc | $-(CH_2)_4-$ | 1 | | $C_7H_{15}$ |
| 19 | $C_2H_5$ | | | 0 | cyc | $-(CH_2)_4-$ | 1 | | $OC_3H_7$ |
| 20 | $C_3H_7$ | | | 0 | cyc | - | 2 | | $CH_3$ |
| 21 | $C_4H_9$ | | | 0 | cyc | - | 2 | | $C_2H_5$ |
| 22 | $CH_3$ | | | 0 | cyc | - | 2 | | $C_4H_9$ |
| 23 | $C_3H_7$ | | | 0 | cyc | - | 2 | | $OCH_3$ |

| | | | | | | | | |
|---|---|---|---|---|---|---|---|---|
| 24 | $C_3H_7$ | phe | - | 1 | cyc | - | 1 | $CH_3$ |
| 25 | $OC_2H_5$ | phe | - | 1 | cyc | - | 1 | $C_7H_{15}$ |
| 26 | $C_2H_5$ | phe | $-OCH_2-$ | 1 | cyc | - | 1 | $C_3H_7$ |
| 27 | $C_4H_9O$ | phe | $-OCH_2-$ | 1 | cyc | - | 1 | $C_{10}H_{21}$ |
| 28 | $C_3H_7$ | | | 0 | phe | - | 1 | $CH_3$ |
| 29 | $C_2H_5O$ | | | 0 | phe | - | 1 | $C_2H_5$ |
| 30 | $CH_3$ | | | 0 | phe | $-CF_2O-$ | 1 | $C_4H_9$ |
| 31 | $C_8H_{17}$ | | | 0 | phe | $-CF_2O-$ | 1 | $OC_2H_5$ |
| 32 | $C_6H_{13}$ | | | 0 | phe | - | 2 | $C_5H_{11}$ |
| 33 | $OC_2H_5$ | | | 0 | phe | - | 2 | $C_2H_5$ |
| 34 | $CH_3$ | phe | $-C\equiv C-$ | 1 | phe | - | 1 | $C_7H_{15}$ |
| 35 | $C_4H_9O$ | phe | $-C\equiv C-$ | 1 | phe | - | 1 | $C_3H_7$ |
| 36 | $C_3H_7$ | phe | $-C\equiv C-$ | 1 | phe | $-(CH_2)_2-$ | 1 | $C_2H_5$ |
| 37 | $C_7H_{15}$ | phe | $-C\equiv C-$ | 1 | phe | $-(CH_2)_4-$ | 1 | $OCH_3$ |
| 38 | $C_2H_5O$ | | | 0 | 2F3Fphe | - | 1 | $C_2H_5$ |
| 39 | $C_3H_7O$ | | | 0 | 2F3Fphe | - | 1 | $OCH_3$ |
| 40 | $CH_3O$ | | | 0 | 2F3Fphe | - | 1 | $C_4H_9$ Cr 60.4 Is |
| 41 | $C_4H_9O$ | | | 0 | 2F3Fphe | - | 1 | $OC_5H_{11}$ |
| 42 | $C_2H_5O$ | 2F3Fphe | - | 1 | cyc | - | 1 | $C_2H_5$ |
| 43 | $C_3H_7O$ | 2F3Fphe | - | 1 | cyc | - | 1 | $C_6H_{13}$ |
| 44 | $C_6H_{13}$ | 2F3Fphe | - | 1 | cyc | - | 1 | $OCH_3$ |
| 45 | $C_2H_5O$ | 2F3Fphe | $-OCH_2-$ | 1 | cyc | - | 1 | $OC_3H_7$ |
| 46 | $CH_3O$ | 2F3Fphe | $-O(CH_2)_3-$ | 1 | cyc | - | 1 | $C_5H_{11}$ |
| 47 | $C_3H_7O$ | 2F3Fphe | $-OCF_2-$ | 1 | cyc | - | 1 | $C_3H_7$ |
| 48 | $CH_3$ | | | 0 | 3F5Fphe | - | 1 | $OC_2H_5$ |
| 49 | F | | | 0 | 3F5Fphe | - | 1 | $C_4H_9$ |
| 50 | F | | | 0 | 3F5Fphe | - | 2 | $OC_3H_7$ |
| 51 | $CF_3$ | | | 0 | 3F5Fphe | - | 1 | $C_7H_{15}$ |
| 52 | $CF_3$ | | | 0 | 3F5Fphe | - | 2 | $CH_3$ |
| 53 | $OCF_3$ | | | 0 | 3F5Fphe | - | 1 | $C_5H_{11}$ |
| 54 | $OCF_3$ | | | 0 | 3F5Fphe | - | 2 | $C_2H_5$ |
| 55 | F | | | 0 | 3Fphe | - | 1 | $C_3H_7$ |
| 56 | F | 3F5Fphe | - | 1 | 3Fphe | - | 1 | $C_6H_{13}$ |

Example 2

Synthesis of 5-(4-(4-trans-pentylcyclohexyl)phenyl)-2-ethylfuran

No. 57

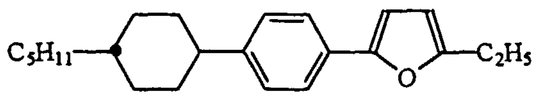

Step 2.1

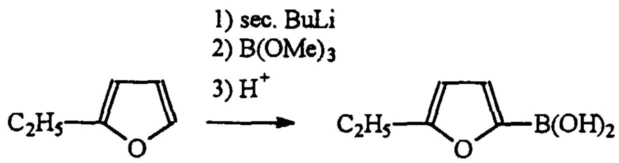


I

II

To a solution of compound I (0.5 mol) in 500 ml of THF, 0.5 mol of sec. BuLi as a solution in n-Hexane is added at -60°C. After stirring for 1 hour at the same temperature a solution of trimethylborate (0.55 mol) in 100 ml of THF is added at a temperature between -60 and -50°C. The reaction mixture is allowed to warm up to room temperature and 100 ml of 6 N aqueous solution of HCl are added. The mixture is stirred for 20 min at room temperature. The organic layer is separated and the aqueous layer is extracted with acetic ester. Evaporation of the solvent gives the crude product II, which can be purified by washing with n-heptane.

Step 2.2

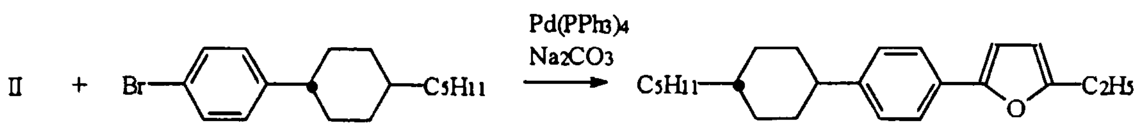


III

IV

To a solution of compound III (0.3 mol) in 200 ml of 1,2-dimethoxyethane, 0.006 mol of $Pd(PPh_3)_4$ is added and stirred for 10 min at room temperature. A solution of compound II (0.3 mol) in 200 ml of THF and a solution of $Na_2CO_3$ (0.45 mol) in 200 ml of water are added. The mixture is refluxed for 6 hours. Compound IV is extracted with toluene. After evaporation of the solvent, compound IV is recrystallized from ethanol. SmA 52 N 60 Is

The following analogous compounds,
No. 58 to 186,

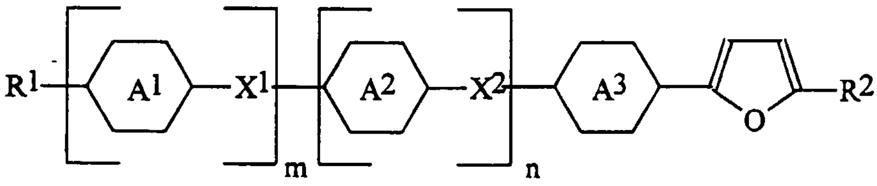

are synthesized.

Table 2

| No. | $R^1$ | $A^1$ | $X^1$ | m | $A^2$ | $X^2$ | n | $A^3$ | 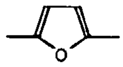 | $R^2$ |
|---|---|---|---|---|---|---|---|---|---|---|
| 58 | $C_5H_{11}$ | | | 0 | | | 0 | phe | | $CH_3$ |
| 59 | $C_3H_7$ | | | 0 | | | 0 | phe | | $C_4H_9$ |
| 60 | $C_2H_5$ | | | 0 | | | 0 | phe | | allyl |
| 61 | $C_4H_9$ | | | 0 | | | 0 | phe | | $OCH_3$ |
| 62 | 3butenyl | | | 0 | | | 0 | phe | | $C_2H_5$ |

| | | | | | | | |
|---|---|---|---|---|---|---|---|
| 63 | allyl | 0 | | | 0 | phe | $OCH_3$ |
| 64 | $C_5H_{11}$ | 0 | cyc | - | 1 | phe | $CH_3$ |
| 65 | $C_3H_7$ | 0 | cyc | - | 1 | phe | 3butenyl |
| 66 | $C_8H_{17}$ | 0 | cyc | - | 1 | phe | $C_4H_9$ |
| 67 | $C_5H_{11}$ | 0 | cyc | - | 1 | phe | $OCH_3$ |
| 68 | vinyl | 0 | cyc | - | 1 | phe | $C_7H_{15}$ |
| 69 | 3butenyl | 0 | cyc | - | 1 | phe | $C_2H_5$ |
| 70 | $C_5H_{11}$ | 0 | phe | - | 1 | phe | $CH_3$ |
| 71 | $C_6H_{13}$ | 0 | phe | - | 1 | phe | $C_3H_7$ |
| 72 | $C_3H_7$ | 0 | phe | - | 1 | phe | vinyl |
| 73 | $C_5H_{11}$ | 0 | phe | - | 1 | phe | $OCH_3$ |
| 74 | vinyl | 0 | phe | - | 1 | phe | $C_9H_{19}$ |
| 75 | 3butenyl | 0 | phe | - | 1 | phe | $C_2H_5$ |
| 76 | $C_5H_{11}$ | 0 | phe | -C≡C- | 1 | phe | $CH_3$ |
| 77 | $C_3H_7$ | 0 | phe | -C≡C- | 1 | phe | 3butenyl |
| 78 | $C_2H_5$ | 0 | phe | -C≡C- | 1 | phe | $C_{10}H_{21}$ |
| 79 | $C_5H_{11}$ | 0 | phe | -C≡C- | 1 | phe | $OCH_3$ |
| 80 | vinyl | 0 | phe | -C≡C- | 1 | phe | $C_4H_9$ |
| 81 | 3butenyl | 0 | phe | -C≡C- | 1 | phe | $C_2H_5$ |
| 82 | $CH_3$ | 0 | phe | $-(CH_2)_2-$ | 1 | phe | $CH_3$ |
| 83 | $C_6H_{13}$ | 0 | phe | $-(CH_2)_2-$ | 1 | phe | $C_2H_5$ |
| 84 | $C_3H_7$ | 0 | phe | $-(CH_2)_2-$ | 1 | phe | $C_4H_9$ |
| 85 | $C_5H_{11}$ | 0 | phe | $-(CH_2)_2-$ | 1 | phe | $OCH_3$ |
| 86 | vinyl | 0 | phe | $-(CH_2)_2-$ | 1 | phe | $C_4H_9$ |
| 87 | 3butenyl | 0 | phe | $-(CH_2)_2-$ | 1 | phe | $C_2H_5$ |
| 88 | $C_5H_{11}$ | 0 | phe | $-(CH_2)_4-$ | 1 | phe | $CH_3$ |
| 89 | $C_3H_7$ | 0 | phe | $-(CH_2)_4-$ | 1 | phe | vinyl |
| 90 | $C_3H_7$ | 0 | phe | $-(CH_2)_4-$ | 1 | phe | $C_4H_9$ |
| 91 | $C_5H_{11}$ | 0 | phe | $-(CH_2)_4-$ | 1 | phe | $OCH_3$ |
| 92 | vinyl | 0 | phe | $-(CH_2)_4-$ | 1 | phe | $C_4H_9$ |
| 93 | 3butenyl | 0 | phe | $-(CH_2)_4-$ | 1 | phe | $C_2H_5$ |
| 94 | $C_2H_5O$ | 0 | | | 0 | 2F3Fphe | $CH_3$ |
| 95 | $C_5H_{11}O$ | 0 | | | 0 | 2F3Fphe | $C_2H_5$ |
| 96 | allylO | 0 | | | 0 | 2F3Fphe | $C_7H_{15}$ |
| 97 | $C_2H_5O$ | 0 | | | 0 | 2F3Fphe | $OCH_3$ |
| 98 | $C_2H_5O$ | 0 | | | 0 | 2Fphe | $C_5H_{11}$ |

| | | | | | | | |
|---|---|---|---|---|---|---|---|
| 99 | $C_2H_5O$ | 0 | | | 0 | 3Fphe | $C_2H_5$ |
| 100 | $C_5H_{11}$ | 0 | cyc | - | 1 | 2F3Fphe | $CH_3$ |
| 101 | $C_3H_7$ | 0 | cyc | - | 1 | 2F3Fphe | $C_2H_5$ |
| 102 | $C_3H_7$ | 0 | cyc | - | 1 | 2F3Fphe | $C_7H_{15}$ |
| 103 | $C_5H_{11}$ | 0 | cyc | - | 1 | 2F3Fphe | $OC_3H_7$ |
| 104 | vinyl | 0 | cyc | - | 1 | 2F3Fphe | $C_5H_{11}$ |
| 105 | 3butenyl | 0 | cyc | - | 1 | 2F3Fphe | $C_3H_7$ |
| 106 | $C_5H_{11}$ | 0 | cyc | - | 1 | 2Fphe | $CH_3$ |
| 107 | $C_3H_7$ | 0 | cyc | - | 1 | 2Fphe | 3butenyl |
| 108 | $C_3H_7$ | 0 | cyc | - | 1 | 2Fphe | $C_6H_{13}$ |
| 109 | $C_9H_{19}$ | 0 | cyc | - | 1 | 2Fphe | $OCH_3$ |
| 110 | vinyl | 0 | cyc | - | 1 | 2Fphe | $C_4H_9$ |
| 111 | 3butenyl | 0 | cyc | - | 1 | 2Fphe | $C_9H_{19}$ |
| 112 | $C_5H_{11}$ | 0 | cyc | - | 1 | 3Fphe | $C_2H_5$ |
| 113 | $C_3H_7$ | 0 | cyc | - | 1 | 3Fphe | 3butenyl |
| 114 | $C_3H_7$ | 0 | cyc | - | 1 | 3Fphe | $OCH_3$ |
| 115 | $C_5H_{11}$ | 0 | cyc | - | 1 | 3Fphe | $OC_5H_{11}$ |
| 116 | vinyl | 0 | cyc | - | 1 | 3Fphe | $C_4H_9$ |
| 117 | 3butenyl | 0 | cyc | - | 1 | 3Fphe | $C_7H_{15}$ |
| 118 | $C_5H_{11}$ | 0 | phe | - | 1 | 2F3Fphe | $CH_3$ |
| 119 | $C_3H_7$ | 0 | phe | - | 1 | 2F3Fphe | allyl |
| 120 | $C_3H_7$ | 0 | phe | - | 1 | 2F3Fphe | $C_8H_{17}$ |
| 121 | $C_5H_{11}$ | 0 | phe | - | 1 | 2F3Fphe | $OCH_3$ |
| 122 | vinyl | 0 | phe | - | 1 | 2F3Fphe | $OC_4H_9$ |
| 123 | 3butenyl | 0 | phe | - | 1 | 2F3Fphe | $C_2H_5$ |
| 124 | $C_5H_{11}$ | 0 | 2Fphe | - | 1 | 2F3Fphe | $CH_3$ |
| 125 | $C_3H_7$ | 0 | 2Fphe | - | 1 | 2F3Fphe | $C_3H_7$ |
| 126 | $C_3H_7$ | 0 | 2Fphe | - | 1 | 2F3Fphe | $C_6H_{13}$ |
| 127 | $C_5H_{11}$ | 0 | 2Fphe | - | 1 | 2F3Fphe | $OCH_3$ |
| 128 | vinyl | 0 | 2Fphe | - | 1 | 2F3Fphe | $C_4H_9$ |
| 129 | 3butenyl | 0 | 2Fphe | - | 1 | 2F3Fphe | $C_2H_5$ |
| 130 | $C_3H_7O$ | 0 | 2Fphe | - | 1 | 2F3Fphe | $OCH_3$ |
| 131 | $CH_3O$ | 0 | 3Fphe | - | 1 | 2F3Fphe | $OC_2H_5$ |
| 132 | $C_5H_{11}O$ | 0 | 2F3Fphe | - | 1 | 2F3Fphe | $OC_6H_{13}$ |
| 133 | $C_2H_5O$ | 0 | 2Fphe | - | 1 | 2Fphe | $OC_3H_7$ |
| 134 | $C_3H_7$ | 0 | 2Fphe | - | 1 | 3Fphe | $OCH_3$ |

| | | | | | | | |
|---|---|---|---|---|---|---|---|
| 135 | $C_3H_7O$ | 0 | 3Fphe | - | 1 | 2Fphe | $OC_4H_9$ |
| 136 | $C_2H_5O$ | 0 | 2F3Fphe | - | 1 | 2Fphe | $OC_7H_{15}$ |
| 137 | $CH_3O$ | 0 | 2F3Fphe | - | 1 | 3Fphe | $OCH_3$ |
| 138 | F | 0 | 3Fphe | - | 1 | phe | $C_2H_5$ |
| 139 | F | 0 | 3F5Fphe | - | 1 | phe | $C_4H_9$ |
| 140 | $CF_3$ | 0 | 3Fphe | - | 1 | phe | $C_3H_7$ |
| 141 | $CF_3$ | 0 | 3F5Fphe | - | 1 | phe | $C_2H_5$ |
| 142 | $OCF_3$ | 0 | 3Fphe | - | 1 | phe | $C_8H_{17}$ |
| 143 | $OCF_3$ | 0 | 3F5Fphe | - | 1 | phe | $C_3H_7$ |
| 144 | F | 0 | 3Fphe | - | 1 | 3Fphe | $C_2H_5$ |
| 145 | F | 0 | 3F5Fphe | - | 1 | 3Fphe | $C_4H_9$ |
| 146 | $CF_3$ | 0 | 3Fphe | - | 1 | 3Fphe | $C_2H_5$ |
| 147 | $CF_3$ | 0 | 3F5Fphe | - | 1 | 3Fphe | $C_5H_{11}$ |
| 148 | $OCF_3$ | 0 | 3Fphe | - | 1 | 3Fphe | $C_2H_5$ |
| 149 | $OCF_3$ | 0 | 3F5Fphe | - | 1 | 3Fphe | $OC_2H_5$ |
| 150 | F | 0 | 3Fphe | - | 1 | 3F5Fphe | $C_2H_5$ |
| 151 | F | 0 | 3F5Fphe | - | 1 | 3F5Fphe | $OC_3H_7$ |
| 152 | $CF_3$ | 0 | 3Fphe | - | 1 | 3F5Fphe | $C_4H_9$ |
| 153 | $CF_3$ | 0 | 3F5Fphe | - | 1 | 3F5Fphe | $OC_8H_{17}$ |
| 154 | $OCF_3$ | 0 | 3Fphe | - | 1 | 3F5Fphe | $C_2H_5$ |
| 155 | $OCF_3$ | 0 | 3F5Fphe | - | 1 | 3F5Fphe | $C_7H_{15}$ |
| 156 | F | 0 | 3Fphe | $-OCF_2-$ | 1 | phe | $C_2H_5$ |
| 157 | F | 0 | 3F5Fphe | $-OCF_2-$ | 1 | phe | $C_2H_5$ |
| 158 | F | 0 | 3Fphe | $-OCF_2-$ | 1 | 3Fphe | $C_2H_5$ |
| 159 | F | 0 | 3F5Fphe | $-OCF_2-$ | 1 | 3Fphe | $C_2H_5$ |
| 160 | CN | 0 | 3Fphe | $-OCF_2-$ | 1 | phe | $C_2H_5$ |
| 161 | CN | 0 | 3F5Fphe | $-OCF_2-$ | 1 | phe | $C_2H_5$ |
| 162 | CN | 0 | 3Fphe | $-OCF_2-$ | 1 | 3Fphe | $C_2H_5$ |
| 163 | CN | 0 | 3F5Fphe | $-OCF_2-$ | 1 | 3Fphe | $C_2H_5$ |
| 163 | $C_5H_{11}$ | 0 | | | 0 | 2pyr | $CH_3$ |
| 164 | $C_3H_7$ | 0 | | | 0 | 2pyr | $C_2H_5$ |
| 165 | $C_5H_{11}$ | 0 | | | 0 | 2pyr | $OCH_3$ |
| 166 | 3butenyl | 0 | | | 0 | 2pyr | $C_2H_5$ |
| 167 | $C_5H_{11}$ | 0 | | | 0 | 3pyr | $CH_3$ |
| 168 | $C_3H_7$ | 0 | | | 0 | 3pyr | $C_2H_5$ |
| 169 | $C_5H_{11}$ | 0 | | | 0 | 3pyr | $OCH_3$ |

| | | | | | | | | | |
|---|---|---|---|---|---|---|---|---|---|
| 170 | 3butenyl | | | 0 | | | 0 | 3pyr | $C_2H_5$ |
| 171 | $OC_2H_5$ | | | 0 | | | 0 | 3F2pyr | $C_2H_5$ |
| 172 | $CH_3$ | | | 0 | | | 0 | 2F3pyr | $C_4H_9$ |
| 173 | $C_3H_7$ | | | 0 | | | 0 | 2,6py | $C_2H_5$ |
| 174 | $C_5H_{11}$ | | | 0 | | | 0 | 2,6py | $OCH_3$ |
| 175 | $C_3H_7$ | | | 0 | cyc | - | 1 | 2,6py | $C_2H_5$ |
| 176 | $C_5H_{11}$ | | | 0 | cyc | - | 1 | 2,6py | $OCH_3$ |
| 177 | $C_3H_7$ | | | 0 | phe | - | 1 | 2,6py | $C_2H_5$ |
| 178 | $C_5H_{11}$ | | | 0 | phe | - | 1 | 2,6py | $OCH_3$ |
| 179 | $C_3H_7$ | | | 0 | cyc | - | 2 | phe | $CH_3$ |
| 180 | $C_3H_7$ | cyc | - | 1 | cyc | $-(CH_2)_2-$ | 1 | phe | $CH_3$ |
| 181 | $C_3H_7$ | | | 0 | cyc | $-(CH_2)_2-$ | 2 | phe | $CH_3$ |
| 182 | $C_3H_7$ | | | 0 | phe | -C≡C- | 2 | phe | $CH_3$ |
| 183 | $C_3H_7$ | 3Fphe | - | 1 | phe | -C≡C- | 1 | phe | $CH_3$ |
| 184 | F | 3Fphe | - | 1 | phe | -C≡C- | 1 | phe | $CH_3$ |
| 185 | F | 3F5Fphe | - | 1 | 3Fphe | - | 1 | phe | $CH_3$ |
| 186 | $C_3H_7$ | cyc | $-(CH_2)_2-$ | 1 | phe | - | 1 | phe | $CH_3$ |

Example 3

Synthesis of 5-(trans-4-propylcyclohexyl)fur-2-yl-3,4-difluorobenzene

No. 187

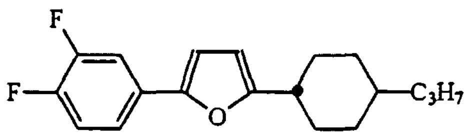

Step 3.1

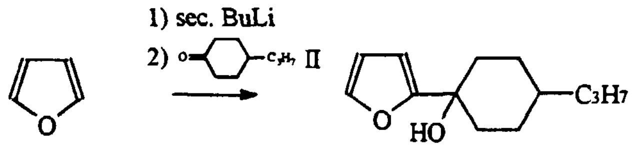


I III

The synthesis of compound III is carried out similarly to step 1.1.

Step 3.2

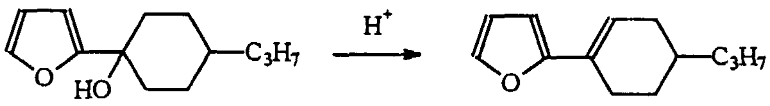


III IV

The synthesis of compound IV is carried out similarly to step 1.2.

Step 3.3

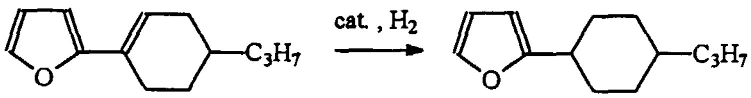


IV V

The synthesis of compound V is carried out similarly to step 1.3.

Step 3.4

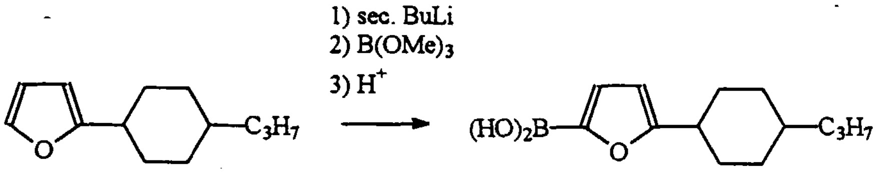


V VI

The synthesis of compound VI is carried out similarly to step 2.1.

Step 3.5

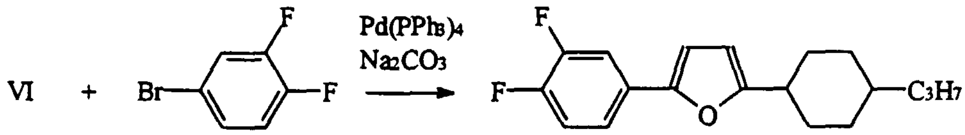


VII VIII

The synthesis of compound VIII is carried out similarly to step 2.2.

Step 3.6

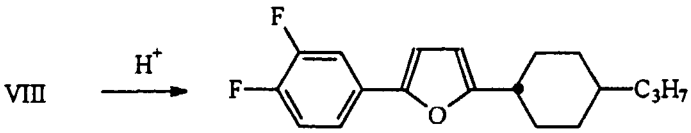


IX

The synthesis of compound IX is carried out similarly to step 1.4.

The following analogous compounds,
No. 188 to 249,

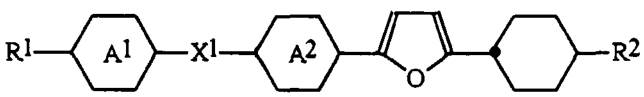

are synthesized.

Table 3

| No. | $R^1$ | $A^1$ | $X^1$ | $A^2$ | 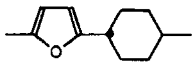 | $R^2$ |
|---|---|---|---|---|---|---|
| 188 | $CH_3$ | | | Phe | | $C_5H_{11}$ |
| 189 | $C_2H_5$ | | | Phe | | $C_2H_5$ |
| 190 | $C_2H_5O$ | | | Phe | | $C_6H_{13}$ |
| 191 | allyl | | | Phe | | $CH_3$ |
| 192 | allylO | | | Phe | | $C_3H_7$ |
| 193 | $CF_3$ | | | Phe | | $C_4H_9$ |
| 194 | $OCF_3$ | | | Phe | | $C_{10}H_{21}$ |
| 195 | CN | | | Phe | | $C_3H_7$ |

| | | | | | |
|---|---|---|---|---|---|
| 196 | F | | | Phe | $C_2H_5$ |
| 197 | Cl | | | Phe | $C_7H_{15}$ |
| 198 | $C_3H_7$ | | | Phe | vinyl |
| 199 | $C_2H_5O$ | | | Phe | 3butenyl |
| 200 | $CF_3$ | | | 3Fphe | $C_3H_7$ |
| 201 | $CF_3O$ | | | 3Fphe | $CH_3$ |
| 202 | CN | | | 3Fphe | $C_4H_9$ |
| 203 | F | | | 3Fphe | $C_3H_7$ |
| 204 | $CF_3$ | | | 3F5Fphe | $C_5H_{11}$ |
| 205 | $CF_3O$ | | | 3F5Fphe | $C_2H_5$ |
| 206 | CN | | | 3F5Fphe | $C_3H_7$ |
| 207 | F | | | 3F5Fphe | $C_3H_7$ Cr 46.8 Is |
| 208 | $CH_3O$ | | | 2F3Fphe | $C_5H_{11}$ |
| 209 | $C_2H_5O$ | | | 2F3Fphe | $C_2H_5$ |
| 210 | $C_3H_7O$ | | | 2F3Fphe | 3butenyl |
| 211 | $CH_3O$ | | | 3F2pyr | $CH_3$ |
| 212 | $C_2H_5O$ | | | 3F2pyr | $C_3H_7$ |
| 213 | $C_5H_{11}O$ | | | 3F2pyr | 3butenyl |
| 214 | $C_3H_7$ | | | py | $C_3H_7$ |
| 215 | $C_3H_7$ | | | py | $C_6H_{13}$ |
| 216 | $C_3H_7$ | | | py | allyl |
| 217 | $C_2H_5$ | cyc | - | phe | $C_3H_7$ |
| 218 | $C_3H_7$ | cyc | - | phe | 3butenyl |
| 219 | $C_2H_5$ | cyc | - | 2Fphe | $C_5H_{11}$ |
| 220 | $C_7H_{15}$ | cyc | - | 2Fphe | vinyl |
| 221 | $C_2H_5$ | phe | - | phe | $C_3H_7$ |
| 222 | $C_6H_{13}$ | phe | - | phe | 3butenyl |
| 223 | $C_2H_5$ | phe | - | 2Fphe | $C_2H_5$ |
| 224 | $C_5H_{11}$ | phe | - | 2Fphe | 4pentenyl |
| 225 | $C_2H_5$ | phe | -C≡C- | phe | $C_3H_7$ |
| 226 | $C_4H_9$ | phe | -C≡C- | phe | 3butenyl |

| | | | | | |
|---|---|---|---|---|---|
| 227 | $C_2H_5$ | phe | $-(CH_2)_2-$ | phe | $C_3H_7$ |
| 228 | $C_{10}H_{21}$ | phe | $-(CH_2)_2-$ | phe | vinyl |
| 229 | $CF_3$ | 3Fphe | - | 3Fphe | $C_3H_7$ |
| 230 | $OCF_3$ | 3Fphe | - | 3Fphe | $C_7H_{15}$ |
| 231 | CN | 3Fphe | - | 3Fphe | $C_3H_7$ |
| 232 | F | 3Fphe | - | 3Fphe | $C_3H_7$ |
| 233 | $CF_3$ | 3F5Fphe | - | 3Fphe | $C_3H_7$ |
| 234 | $OCF_3$ | 3F5Fphe | - | 3Fphe | $C_3H_7$ |
| 235 | CN | 3F5Fphe | - | 3Fphe | $C_3H_7$ |
| 236 | F | 3F5Fphe | - | 3Fphe | $C_3H_7$ |
| 237 | F | 3F5Fphe | $-OCF_2-$ | 3Fphe | $C_3H_7$ |
| 238 | $OCH_3$ | 2F3Fphe | - | phe | $C_5H_{11}$ |
| 239 | $OC_2H_5$ | 2F3Fphe | - | phe | $C_3H_7$ |
| 240 | $OC_2H_5$ | 2F3Fphe | - | phe | 3butenyl |
| 241 | $OCH_3$ | 3F2pyr | - | phe | $C_5H_{11}$ |
| 242 | $OC_2H_5$ | 3F2pyr | - | phe | $C_3H_7$ |
| 243 | $OC_2H_5$ | 3F2pyr | - | phe | 3butenyl |
| 244 | $OCH_3$ | 2F3Fphe | - | 3Fphe | $C_5H_{11}$ |
| 245 | $OC_2H_5$ | 2F3Fphe | - | 3Fphe | $C_3H_7$ |
| 246 | $OC_2H_5$ | 2F3Fphe | - | 3Fphe | 3butenyl |
| 247 | $OCH_3$ | 2F3Fphe | - | 2F3Fphe | $C_5H_{11}$ |
| 248 | $OC_2H_5$ | 2F3Fphe | - | 2F3Fphe | $C_3H_7$ |
| 249 | $OC_2H_5$ | 2F3Fphe | - | 2F3Fphe | 3butenyl |

Example 4

Synthesis of 2-(4-trans-pentylcyclohexyl)-5-(4-trans-propylcyclohexyl)furan

No. 250

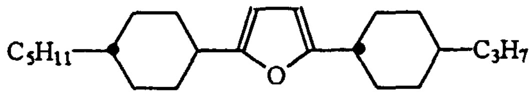

Step 4.1

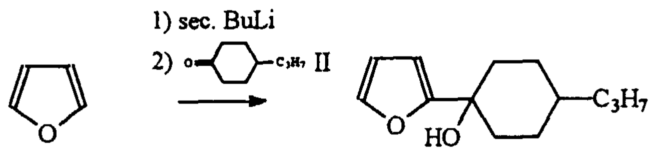


I III

The synthesis of compound III is carried out similarly to step 1.1.

Step 4.2

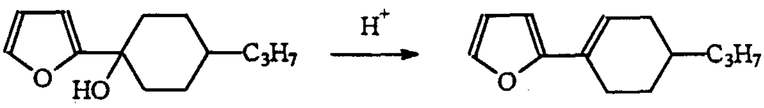


III IV

The synthesis of compound IV is carried out similarly to step 1.2.

Step 4.3

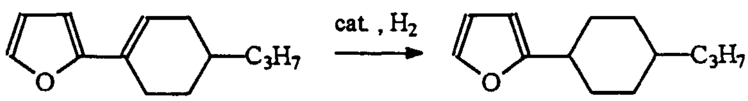


IV V

The synthesis of compound V is carried out similarly to step 1.3.

Step 4.4

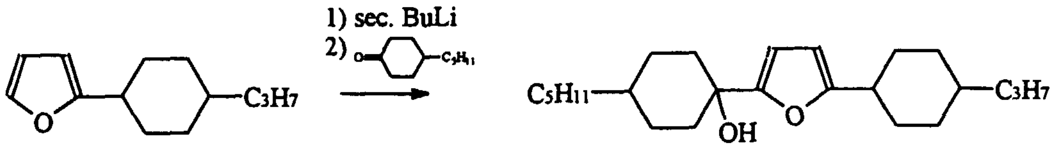


V VI

The synthesis of compound VI is carried out similarly to step 1.1.

Step 4.5

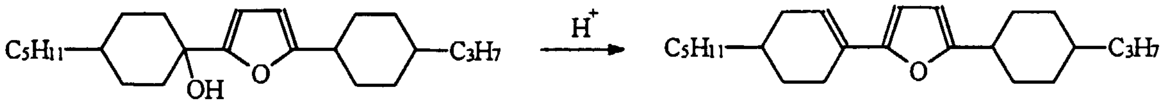


VI VII

The synthesis of compound VII is carried out similarly to step 1.2.

Step 4.6

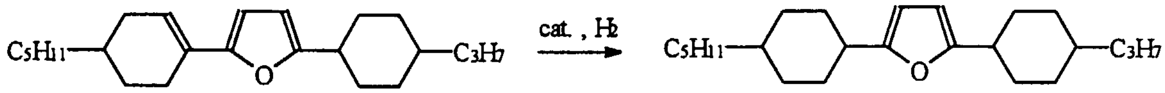


VII VIII

The synthesis of compound VIII is carried out similarly to step 1.3.

Step 4.7

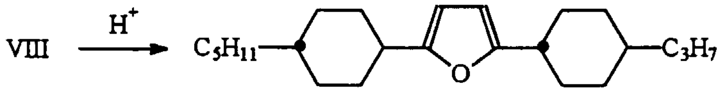

IX

The synthesis of compound IX is carried out similarly to step 1.4. Compound IX is subjected to column chromatography with n-heptane as eluent and recrystallized from ethanol and heptane.

The following analogous compounds,

No. 251 to 272,

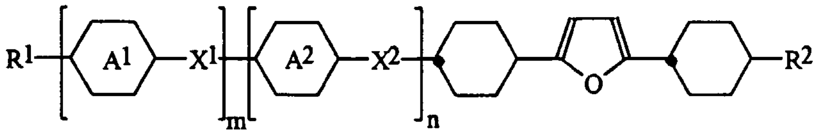

are synthesized.

Table 4

| No. | $R^1$ | $A^1$ | $X^1$ | m | $A^2$ | $X^2$ | n | 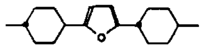 | $R^2$ |
|---|---|---|---|---|---|---|---|---|---|
| 251 | $CH_3$ | | | 0 | | | 0 | | $C_3H_7$ |
| 252 | 3butenyl | | | 0 | | | 0 | | $C_2H_5$ |
| 253 | $CH_3O$ | | | 0 | | | 0 | | $C_5H_{11}$ |
| 254 | $C_3H_7$ | | | 0 | cyc | - | 1 | | $C_3H_7$ |
| 255 | $C_5H_{11}$ | | | 0 | cyc | - | 1 | | $CH_3$ |
| 256 | $C_3H_7$ | | | 0 | cyc | - | 2 | | $C_8H_{17}$ |
| 257 | $C_4H_9$ | | | 0 | cyc | $-(CH_2)_2-$ | 1 | | $C_3H_7$ |
| 258 | $C_2H_5$ | | | 0 | cyc | $-(CH_2)_4-$ | 1 | | $C_6H_{13}$ |
| 259 | $C_3H_7$ | | | 0 | phe | - | 1 | | $C_3H_7$ |
| 260 | $C_2H_5O$ | | | 0 | phe | - | 1 | | vinyl |
| 261 | F | | | 0 | 3Fphe | - | 1 | | $C_5H_{11}$ |
| 262 | $CF_3O$ | | | 0 | 3Fphe | - | 1 | | $C_3H_7$ |
| 263 | $CF_3$ | | | 0 | 3Fphe | - | 1 | | $C_2H_5$ |
| 264 | F | | | 0 | 3F5Fphe | - | 1 | | 3butenyl |
| 265 | CN | | | 0 | 3F5Fphe | - | 1 | | $C_7H_{15}$ |
| 266 | F | 3F5Fphe | - | 1 | 3Fphe | - | 1 | | $C_4H_9$ |
| 267 | F | 3F5Fphe | - | 1 | 3F5Fphe | - | 1 | | $C_2H_5$ |
| 268 | CN | 3F5Fphe | - | 1 | 3Fphe | - | 1 | | $CH_3$ |
| 269 | $C_2H_5O$ | | | 0 | 2F3Fphe | - | 1 | | $C_3H_7$ |
| 270 | $CH_3O$ | | | 0 | 3F2pyr | - | 1 | | $C_5H_{11}$ |
| 271 | $C_2H_5O$ | | | 0 | 2F3Fphe | - | 2 | | $C_3H_7$ |
| 272 | $C_4H_9O$ | 2F3Fphe | - | 1 | 2Fphe | - | 1 | | $C_2H_5$ |

Example 5

Synthesis of 2-(4-trans-(2,3-difluoro-4-methoxyphenyl)-cyclohexyl)-5-(5-trans-propyl-1,3-dioxan-2-yl)furan

No. 273

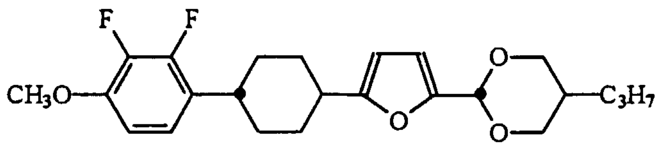

Step 5.1

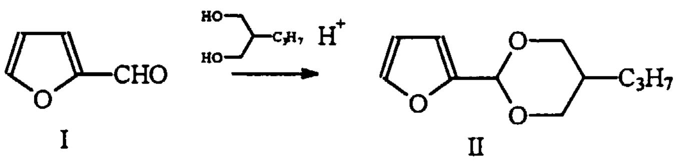


I

II

A solution of compound I (0.5 mol), 2-propylpropane-1,3-diol (0.5 mol) and 4-toluenesulphonic acid (1 g) in 500 ml of toluene is refluxed on a water separator until no more water separates. The solution is washed with a saturated solution of $NaHCO_3$ and with water, and dried over $MgSO_4$. The solvent is evaporated in vacuo and the crude product is subjected to column chromatography with n-heptane as eluent.

Step 5.2

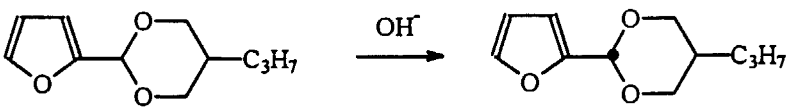


II

III

A solution of compound II (0.45 mol) and sodium hydroxide (2 g) in 300 ml of THF are refluxed for 5 hours. The solvent is evaporated in vacuo and the crude product is dissolved in 300 ml of diethylether. The solution is washed with water and dried over $MgSO_4$. The solvent is distilled off in vacuo. Compound III is obtained by recrystallization from methanol.

Step 5.3

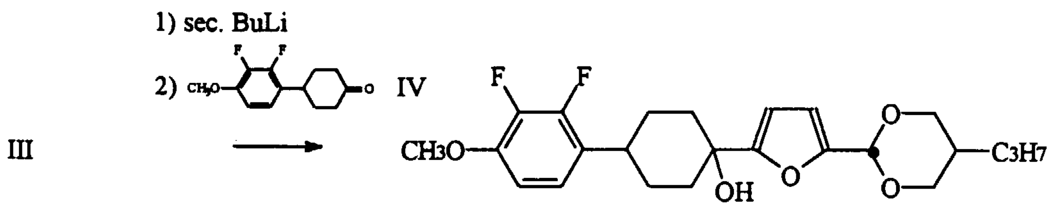


V

The synthesis of compound V is carried out similarly to step 1.1.

Step 5.4

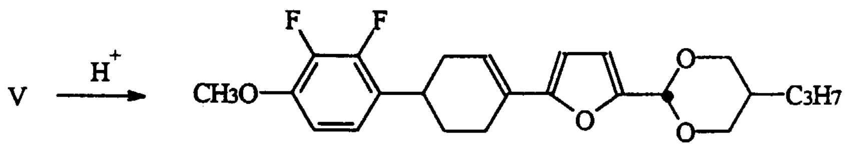


VI

The synthesis of compound VI is carried out similarly to step 1.2.

Step 5.5

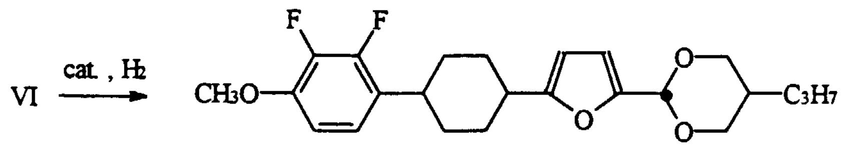


VII

The synthesis of compound VII is carried out similarly to step 1.3.

Step 5.6

VII 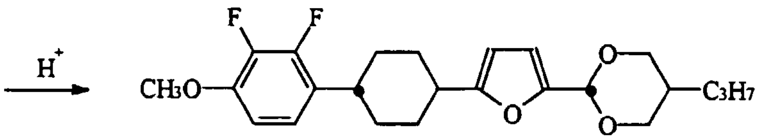


VIII

The synthesis of compound VIII is carried out similarly to step 1.4.

The following analogous compounds,
No. 274 to 289,

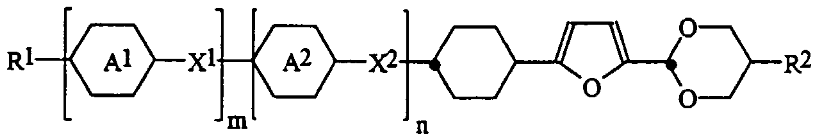

are synthesized.

Table 5

| No. | $R^1$ | $A^1$ | $X^1$ | m | $A^2$ | $X^2$ | n | 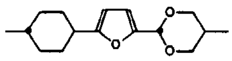 | $R^2$ |
|---|---|---|---|---|---|---|---|---|---|
| 274 | $C_3H_7$ | | | 0 | | | 0 | | $C_5H_{11}$ |
| 275 | $C_4H_9$ | | | 0 | | | 0 | | $C_2H_5$ |
| 276 | $CH_3$ | | | 0 | | | 0 | | $C_9H_{19}$ |
| 277 | $C_5H_{11}$ | | | 0 | cyc | - | 1 | | $C_3H_7$ |
| 278 | CN | | | 0 | phe | - | 1 | | $C_3H_7$ |
| 279 | CN | | | 0 | 3F5Fphe | - | 1 | | $C_4H_9$ |
| 280 | $CF_3$ | | | 0 | 3Fphe | - | 1 | | $C_5H_{11}$ |
| 281 | F | | | 0 | 3Fphe | - | 1 | | $C_3H_7$ |
| 282 | $CF_3O$ | | | 0 | phe | - | 1 | | $C_4H_9$ |
| 283 | $CF_3O$ | 3Fphe | - | 1 | phe | - | 1 | | $C_2H_5$ |
| 284 | F | 3F5Fphe | - | 1 | 3Fphe | - | 1 | | $C_3H_7$ |

| | | | | | | | | |
|---|---|---|---|---|---|---|---|---|
| 285 | $C_2H_5O$ | | | 0 | 2F3Fphe | - | 1 | $C_5H_{11}$ |
| 286 | $C_2H_5O$ | | | 0 | 2F3Fphe | - | 2 | $C_3H_7$ |
| 287 | $CH_3O$ | 2F3Fphe | - | 1 | phe | - | 1 | $C_6H_{13}$ |
| 289 | $C_3H_7$ | cyc | - | 1 | 2F3Fphe | - | 1 | $CH_3$ |

Furthermore, the nematic liquid crystal compositions containing the compounds of the present invention are illustrated in Examples 11 to 21. The compounds in the exemplary compositions are represented by the symbols as shown in Table 6.

Table 6

Representation of compounds by use of the symbols

$R-(A_1)-Z_1-...-Z_n-(A_n)-X$

| 1) Left terminal group R- | Symbol | 3) Bond group $-Z_1-$, $-Z_n-$ | Symbol |
|---|---|---|---|
| $C_nH_{2n+1}-$ | n- | $-C_2H_4-$ | 2 |
| $C_nH_{2n+1}O-$ | nO- | $-C_4H_8-$ | 4 |
| $C_nH_{2n+1}OC_mH_{2m}-$ | nOm- | -COO- | E |
| $CH_2=CH-$ | V- | $-C\equiv C-$ | T |
| $CH_2=CH-C_nH_{2n}-$ | Vn- | -CH=CH- | V |
| $C_nH_{2n+1}CH=CHC_mH_{2m}-$ | nVm- | $-CF_2O-$ | CF2O |
| $C_nH_{2n+1}CH=CHC_mH_{2m}CH=CHC_kH_{2k}-$ | nVmVk- | $-OCF_2-$ | OCF2 |
| **2) Ring structure $-(A_1)-$, $-(A_n)-$** | **Symbol** | **4) Right terminal group -X** | **Symbol** |
| 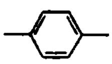 | B | -F<br>-Cl | -F<br>-CL |

| | | | |
|---|---|---|---|
| 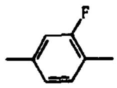 | B(F) | $-CN$ | -C |
| | | $-CF_3$ | -CF3 |
| 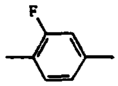 | B(2F) | $-OCF_3$ | -OCF3 |
| | | $-OCF_2H$ | -OCF2H |
| 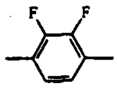 | B(2F,3F) | $-C_nH_{2n+1}$ | -n |
| | | $-OC_nH_{2n+1}$ | -On |
| | | $-COOCH_3$ | -EMe |
| 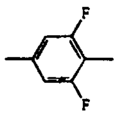 | B(F,F) | $-C_nH_{2n}CH=CH_2$ | -nV |
| | | $-C_mH_{2m}CH=CHC_nH_{2n+1}$ | -mVn |
| | | $-C_mH_{2m}CH=CHC_nH_{2n}F$ | -mVnF |
| 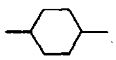 | H | $-CH=CF_2$ | -VFF |
| 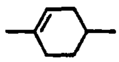 | Ch | $-C_nH_{2n}CH=CF_2$ | -nVFF |
| | | $-C\equiv C-CN$ | -TC |
| 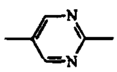 | Py | | |
| 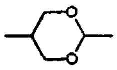 | D | | |
| 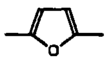 | Y | | |

5) Examples of Representation

| Ex. 1 3-H2B(F,F)B(F)-F | Ex. 2 3-HB(F)TB-2 | Ex. 3 1V2-BEB(F,F)-C |
|---|---|---|
| 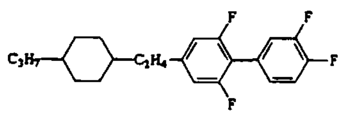 | 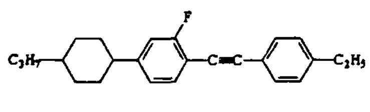 | 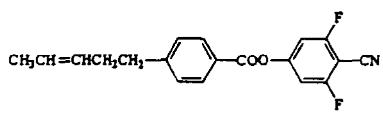 |

(In this table, n, m and k are each an integer.)

Example 6

| | |
|---|---|
| 1-YChH-5 | 15.0% |
| 3-HB-C | 20.4% |

(continued)

| | |
|---|---|
| 5-HB-C | 30.6% |
| 7-HB-C | 21.2% |
| 3-HBB-C | 12.8% |

$T_{NI}$ = 72.5 (°C)
η = 29.4 (mPa • s)
Δn = 0.131
Δε = 10.0
$V_{th}$ = 1.90 (V)

Example 7

| | |
|---|---|
| 1-YHH-5 | 15.0% |
| 3-HB-C | 20.4% |
| 5-HB-C | 30.6% |
| 7-HB-C | 21.2% |
| 3-HBB-C | 12.8% |

$T_{NI}$ = 72.2 (°C)
η = 26.6 (mPa • s)
Δn = 0.126
Δε = 9.9
$V_{th}$ = 1.84 (V)

Example 8

| | |
|---|---|
| 4-YHB(2F,3F)-O1 | 15.0% |
| 3-HEB-O2 | 12.9% |
| 3-HEB-O4 | 20.7% |
| 4-HEB-O2 | 15.5% |
| 5-HEB-O1 | 15.5% |
| 5-HEB-O2 | 10.4% |

$T_{NI}$ = 55.9 (°C)
η = 27.9 (mPa • s)

$\Delta n$ = 0.082
$\Delta\varepsilon$ = -1.83
$V_{th}$ = 1.90 (V)

Example 9

| | |
|---|---|
| 1-YBH-5 | 15.0% |
| 3-HB-C | 20.4% |
| 5-HB-C | 30.6% |
| 7-HB-C | 21.2% |
| 3-HBB-C | 12.8% |

$T_{NI}$ = 68.9 (°C)
$\eta$ = 32.0 (mPa • s)
$\Delta n$ = 0.134
$\Delta\varepsilon$ = 10.2
$V_{th}$ = 1.65 (V)

Example 10

| | |
|---|---|
| 3-HYB(F,F)-F | 15.0% |
| 3-HB-C | 20.4% |
| 5-HB-C | 30.6% |
| 7-HB-C | 21.2% |
| 3-HBB-C | 12.8% |

$T_{NI}$ = 67.4 (°C)
$\eta$ = 27.6 (mPa • s)
$\Delta n$ = 0.124
$\Delta\varepsilon$ = 10.9
$V_{th}$ = 1.63 (V)

Example 11

| | |
|---|---|
| 5-HHY-1 | 10 % |

(continued)

| | |
|---|---|
| 1V2-BEB(F,F)-C | 5 % |
| 3-HB-C | 25 % |
| 1-BTB-3 | 5 % |
| 2-BTB-1 | 10 % |
| 3-HH-4 | 4 % |
| 3-HHB-1 | 11 % |
| 3-HHB-3 | 6 % |
| 3-H2BTB-2 | 4 % |
| 3-H2BTB-3 | 4 % |
| 3-H2HBTB-4 | 4 % |
| 3-HB(F)TB-2 | 6 % |
| 3-HB(F)TB-3 | 6 % |
| CM33 | ratio 0.8 |

$T_{NI}$ = 89.1 (°C)
$\eta$ = 16.8 (mPa • s)
$\Delta n$ = 0.160
$\Delta\varepsilon$ =7.0
$V_{th}$ = 2.09 (V)
P = 10.8 µm

Example 12

| | |
|---|---|
| 5-HHY-1 | 5 % |
| 2O1-BEB(F)-C | 5 % |
| 3O1-BEB(F)-C | 15 % |
| 4O1-BEB(F)-C | 13 % |
| 5O1-BEB(F)-C | 13 % |
| 2-HHB(F)-C | 15 % |
| 3-HHB(F)-C | 15 % |
| 3-HB(F)TB-2 | 4 % |
| 3-HB(F)TB-3 | 4 % |
| 3-HB(F)TB-4 | 4 % |
| 3-HHB-1 | 3 % |
| 3-HHB-O1 | 4 % |

$T_{NI}$ = 86.7 (°C)

$\eta$ = 88.3 (mPa • s)
$\Delta n$ = 0.148
$\Delta\varepsilon$ = 30.9
$V_{th}$ = 0.84 (V)

Example 13

| | |
|---|---|
| 3-HHY-1 | 7 % |
| 5-BEB(F)-C | 5 % |
| V-HB-C | 11 % |
| 5-PyB-C | 6 % |
| 4-BB-3 | 11 % |
| 3-HH-2V | 10 % |
| 5-HH-V | 11 % |
| V-HHB-1 | 7 % |
| V2-HHB-1 | 8 % |
| 3-HHB-1 | 9 % |
| 1V2-HBB-2 | 10 % |
| 3-HHEBH-3 | 5 % |

$T_{NI}$ = 85.5 (°C)
$\eta$ = 15.8 (mPa • s)
$\Delta n$ = 0.113
$\Delta\varepsilon$ = 4.6
$V_{th}$ = 2.32 (V)

Example 14

| | |
|---|---|
| 5-HHY-1 | 6 % |
| 1V2-BEB(F,F)-C | 6 % |
| 3-HB-C | 13 % |
| 3-H[5D,6D,7D]B-C | 5 % |
| 2-BTB-1 | 10 % |
| 5-HH-VFF | 30 % |
| 1-BHH-VFF | 8 % |
| 1-BHH-2VFF | 5 % |
| 3-H2BTB-2 | 5 % |

(continued)

| | |
|---|---|
| 3-H2BTB-3 | 4 % |
| 3-H2BTB-4 | 4 % |
| 3-HHB-1 | 4 % |

$T_{NI}$ = 75.9 (°C)
η = 12.8 (mPa • s)
Δn = 0.127
Δε = 6.2
$V_{th}$ = 2.08 (V)

Example 15

| | |
|---|---|
| 3-HHY-1 | 20 % |
| 5-HHY-1 | 10 % |
| 2-HHB(F)-F | 13 % |
| 3-HHB(F)-F | 13 % |
| 5-HHB(F)-F | 14 % |
| 2-H2HB(F)-F | 4 % |
| 3-H2HB(F)-F | 2 % |
| 5-H2HB(F)-F | 4 % |
| 2-HBB(F)-F | 5 % |
| 3-HBB(F)-F | 5 % |
| 5-HBB(F)-F | 10 % |
| CN | ratio 0.3 |

$T_{NI}$ = 93.9 (°C)
η = 24.9 (mPa • s)
Δn = 0.086
Δε = 3.4
$V_{th}$ = 2.57 (V)
P = 76 μm

Example 16

| | |
|---|---|
| 5-HHY-1 | 8 % |
| 5-HBY-2 | 3 % |

(continued)

| | |
|---|---|
| 3-HB-CL | 10 % |
| 5-HB-CL | 4 % |
| 7-HB-CL | 4 % |
| 1O1-HH-5 | 5 % |
| 2-HBB(F)-F | 8 % |
| 3-HBB(F)-F | 8 % |
| 5-HBB(F)-F | 14 % |
| 4-HHB-CL | 5 % |
| 3-H2HB(F)-CL | 4 % |
| 3-HBB(F,F)-F | 10 % |
| 5-H2BB(F,F)-F | 9 % |
| 3-HB(F)VB-2 | 4 % |
| 3-HB(F)VB-3 | 4 % |

$T_{NI}$ = 82.3 (°C)
η = 22.3 (mPa • s)
Δn = 0.126
Δε = 4.5
$V_{th}$ = 2.39 (V)

Example 17

| | |
|---|---|
| 5-HHY-1 | 6 % |
| 5-HBY-2 | 6 % |
| 3-HB-C | 18 % |
| 7-HB-C | 3 % |
| 1O1-HB-C | 10 % |
| 3-HB(F)-C | 10 % |
| 2-PyB-2 | 2 % |
| 3-PyB-2 | 2 % |
| 4-PyB-2 | 2 % |
| 1O1-NH-3 | 7 % |
| 2-BTB-O1 | 7 % |
| 3-HHB-1 | 7 % |
| 3-HHB-F | 4 % |
| 3-H2BTB-2 | 3 % |
| 3-H2BTB-3 | 3 % |
| 2-PyBH-3 | 4 % |

(continued)

| | |
|---|---|
| 3-PyBH-3 | 3 % |
| 3-PyBB-2 | 3 % |

Example 18

| | |
|---|---|
| 3-HYB(F,F)-F | 10 % |
| 3-HHB(F,F)-F | 9 % |
| 3-H2HB(F,F)-F | 8 % |
| 4-H2HB(F,F)-F | 8 % |
| 3-HHB(F)-OCF3 | 8 % |
| 3-HBB(F,F)-F | 21 % |
| 5-HBB(F,F)-F | 10 % |
| 3-H2BB(F,F)-F | 10 % |
| 5-HHBB(F,F)-F | 3 % |
| 5-HHEBB-F | 2 % |
| 3-HH2BB(F,F)-F | 3 % |
| 1O1-HBBH-4 | 4 % |
| 1O1-HBBH-5 | 4 % |
| CM43L | ratio 0.25 |

Example 19

| | |
|---|---|
| 3-HYB(F,F)-F | 10 % |
| 7-HB(F,F)-F | 5 % |
| 3-H2HB(F,F)-F | 12 % |
| 3-HHB(F,F)-F | 10 % |
| 4-HHB(F,F)-F | 5 % |
| 3-HBB(F,F)-F | 10 % |
| 3-HHEB(F,F)-F | 10 % |
| 4-HHEB(F,F)-F | 3 % |
| 5-HHEB(F,F)-F | 3 % |
| 2-HBEB(F,F)-F | 3 % |
| 3-HBEB(F,F)-F | 5 % |
| 5-HBEB(F,F)-F | 3 % |
| 3-HDB(F,F)-F | 15 % |

(continued)

| | |
|---|---|
| 3-HHBB(F,F)-F | 6 % |

Example 20

| | |
|---|---|
| 3-HYB(2F,3F)-O2 | 10 % |
| 3-HH-2 | 5 % |
| 3-HH-4 | 6 % |
| 3-HH-O1 | 4 % |
| 3-HH-O3 | 5 % |
| 5-HH-O1 | 4 % |
| 3-HB(2F,3F)-O2 | 12 % |
| 5-HB(2F,3F)-O2 | 5 % |
| 3-HHB(2F,3F)-O2 | 10 % |
| 5-HHB(2F,3F)-O2 | 15 % |
| 3-HHB(2F,3F)-2 | 24 % |

Example 21

| | |
|---|---|
| 3-HYB(2F,3F)-O2 | 8 % |
| 3-YBB(2F,3F)-O1 | 8 % |
| 3-BB(2F,3F)-O2 | 10 % |
| 5-BB-5 | 9 % |
| 5-BB-O6 | 5 % |
| 5-BB-O8 | 4 % |
| 1-BEB-5 | 4 % |
| 5-BEB-5 | 3 % |
| 3-HEB-O2 | 20 % |
| 5-BBB(2F,3F)-7 | 9 % |
| 3-H2BB(2F)-5 | 20 % |

## Claims

1. A liquid crystal compound incorporating a furan-2,5-diyl moiety which has nematic, smectic, latent nematic or smectic mesophases and is represented by the formula (1)

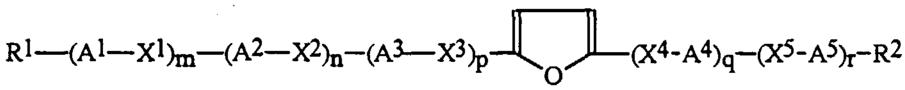

(1)

where $A^1$, $A^2$, $A^3$, $A^4$ and $A^5$ are each independently trans-1,4-cyclohexylene in which one or two methylene group(s) may optionally be substituted with difluoromethylene group(s), 1,4-cyclohexenylene, 1,3-dioxane-2,5-diyl, tetrahydropyran-2,5-diyl in which one methylene group may optionally be substituted with a difluoromethylene group, 1,3-dithiane-2,5-diyl, 1,4-phenylene in which one or more hydrogen atom(s) may optionally be substituted with halogen atom(s) or $-CF_3$ group(s), pyrimidine-2,5-diyl, pyridine-2,5-diyl in which one hydrogen atom may optionally be substituted with a halogen atom, thiazole-2,5-diyl, thiadiazole-2,5-diyl or thiophene-2,5-diyl; $X^1$, $X^2$, $X^3$, $X^4$ and $X^5$ are each independently $-(CH_2)_2-$ or $-(CH_2)_4-$ where one $-CH_2-$ group may be substituted with -O-, -S-, -CH=CH-, -C≡C-, $-CF_2O-$, $-OCF_2-$, $-CF_2S-$ or $-SCF_2-$, or a single bond; m, n, p, q and r are each independently an integer from 0 to 2 with the proviso that $1 \leq m + n + p + q + r \leq 4$; and $R^1$ and $R^2$ are each independently a straight or branched alkyl chain of 1 to 18 carbon atoms in which one or more $-CH_2-$ group(s) may be substituted with -O-, -S-, $-CF_2-$, -CHF-, $-SiH_2-$, $-SiH(CH_3)-$, $-Si(CH_3)_2-$, - CH=CH- or -C≡C- with the proviso that two similar heteroatoms are not neighbored, and the terminal $-CH_3$ group may be substituted with $-CF_3$, $-OCF_3$, $-CF_2H$, $-CH=CH_2$, $-CH=CF_2$, -CN or a halogen atom; provided that $R^1$ shall not be -CN if q = 1, m = n = p = r = 0 , $X^4$ is a single bond, $A^4$ is 1,4-phenylene and $R^2$ is an n-alkyl chain, and that $R^2$ shall not be -CN if p = 1, m = p = q = r = 0 , $X^3$ is a single bond, $A^3$ is 1,4-phenylene and $R^1$ is an n-alkyl chain.

2. A liquid crystal compound of claim 1 represented by the formula (1); where $A^1$, $A^2$, $A^3$, $A^4$ and $A^5$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$, $X^2$, $X^3$, $X^4$ and $X^5$ are each independently - $(CH_2)_2-$ or $-(CH_2)_4-$ in which one $-CH_2-$ group may be substituted with -O-, -C≡C-, $-CF_2O-$ or $-OCF_2-$, or a single bond; m, n, p, q and r are each independently an integer from 0 to 2 with the proviso that $1 \leq m + n + p + q + r \leq 4$; and $R^1$ and $R^2$ are each independently a straight or branched alkyl chain of 1 to 12 carbon atoms in which one or more - $CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

3. A liquid crystal compound of claim 1 represented by the formula (1); where $A^1$, $A^2$ and $A^5$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$, $X^2$ and $X^5$ are each independently $-(CH_2)_2-$ or $-(CH_2)_4-$ in which one $-CH_2-$ group may be substituted with -O-, -C≡C-, $-CF_2O-$ or $-OCF_2-$, or a single bond; m, n and r are each independently an integer from 0 to 2 with the proviso that $m + n + r = 3$ and $p = q = 0$; and $R^1$ and $R^2$ are each independently a straight or branched alkyl chain of 1 to 12 carbon atoms in which one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

4. A liquid crystal compound of claim 1 represented by the formula (1); where $A^1$, $A^2$ and $A^3$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$, $X^2$ and $x^3$ are each independently $-(CH_2)_2-$ or $-(CH_2)_4-$ in which one $-CH_2-$ group may be substituted with -O-, -C≡C-, $-CF_2O-$ or $-OCF_2-$, or a single bond; m, n and p are each independently an integer from 0 to 2 with the proviso that $m + n + p = 3$ and $q = r = 0$; and $R^1$ and $R^2$ are each independently a straight or branched alkyl chain of 1 to 12 carbon atoms in which one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

**5.** A liquid crystal compound of claim 1 represented by the formula (1); where $A^1$ and $A^5$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, pyridine-2,5-diyl in which one hydrogen atom may optionally be substituted with a halogen atom; $X^1$ and $X^5$ are each independently $-(CH_2)_2-$ or $-(CH_2)_4-$ where one $-CH_2-$ group may be substituted with -O-, -C≡C-, $-CF_2O-$ or $-OCF_2-$, or a single bond; m and r are each independently an integer from 0 to 2 with the proviso that $m + r = 2$ and $n = p = q = 0$; and $R^1$ and $R^2$ are each independently a straight or branched alkyl chain of 1 to 12 carbon atoms where one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

**6.** A liquid crystal compound of claim 1 represented by the formula (1); where $A^1$ and $A^2$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$ and $X^2$ are each independently $-(CH_2)_2-$ or $-(CH_2)_4-$ in which one $-CH_2-$ group may be substituted with -O-, -C≡C-, $-CF_2O-$ or $-OCF_2-$, or a single bond; m and n are each independently an integer from 0 to 2 with the proviso that $m + n = 2$ and $p = q = r = 0$; and $R^1$ and $R^2$ are each independently a straight or branched alkyl chain of 1 to 12 carbon atoms where one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$ or a halogen atom selected from fluorine and chlorine.

**7.** A liquid crystal compound of claim 1 represented by the formula (1); where $A^1$, $A^2$ and $A^3$ are each independently trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $A^4$ is trans-1,4-cyclohexylene; $X^1$, $X^2$ and $X^3$ are each independently $-(CH_2)_2-$ or $-(CH_2)_4-$ where one $-CH_2-$group may be substituted with -O-, -C≡C-, $-CF_2O-$ or $-OCF_2-$, or a single bond; $X^4$ is a single bond; m, n and p are each independently an integer from 0 to 2 with the proviso that $1 \leq m + n + p \leq 3$, $q = 1$ and $r = 0$; and $R^1$ and $R^2$ are each independently a straight or branched alkyl chain of 1 to 12 carbon atoms in which one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

**8.** A liquid crystal compound of claim 1 represented by the formula (1); where $A^1$ is trans-1,4-cyclohexylene, 1,3-dioxane-2,5-diyl, 1,4-phenylene in which one or more hydrogen atom(s) may optionally be substituted with halogen atom(s) or $-CF_3$ group(s), pyrimidine-2,5-diyl, or pyridine-2,5-diyl in which one hydrogen atom may optionally be substituted with a halogen atom; $X^1$ is $-(CH_2)_2-$ or $-(CH_2)_4-$where one $-CH_2-$ group may be substituted with -O-, -C≡C-, $-CF_2O-$ or $-OCF_2-$, or a single bond; $m = 1$; $n = p = q = r = 0$; and $R^1$ and $R^2$ are each independently a straight or branched alkyl chain of 1 to 12 carbon atoms in which one or more $-CH_2-$ group(s) may be substituted with -O- or -CH=CH- with the proviso that two oxygen atoms are not neighbored, and the terminal $-CH_3$ group may be substituted with $-CF_3$, $-OCF_3$, $-CF_2H$, $-CH=CH_2$, $-CH=CF_2$, -CN or a halogen atom selected from fluorine and chlorine.

**9.** A liquid crystal composition which comprises two or more components containing at least one liquid crystal compound as claimed in any one of claims 1 to 8.

**10.** A liquid crystal composition which contains at least one liquid crystal compound as claimed in any one of claims 1 to 8 as a first component and at least one compound selected from the group consisting of the formulae (2), (3) and (4) as a second component,

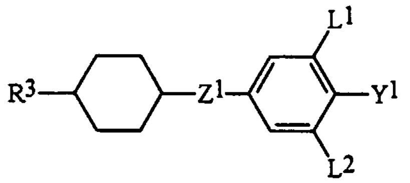

(2)

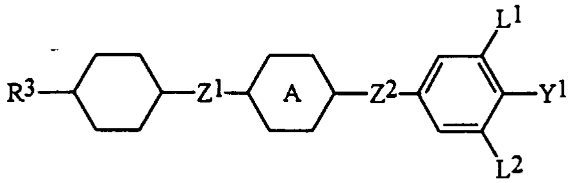

(3)

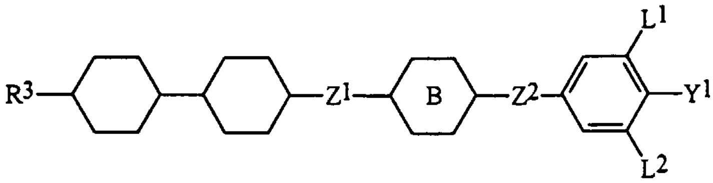

(4)

where $R^3$ is an alkyl group having 1 to 10 carbon atoms in which any unadjacent methylene group may be substituted with an oxygen atom or -CH=CH- and any hydrogen atom may be substituted with a fluorine atom; $Y^1$ is F, Cl, $OCF_3$, $OCF_2H$, $CF_3$, $CF_2H$, $CFH_2$, $OCF_2CF_2H$ or $OCF_2CFHCF_3$; $L^1$ and $L^2$ are each independently H or F; $Z^1$ and $Z^2$ are each independently - $(CH_2)_2$-, -$(CH_2)_4$-, -COO-, -$CF_2O$-, -$OCF_2$-, -CH=CH- or a single bond; ring A is trans-1,4-cyclohexylene, 1,3-dioxane-2,5-diyl or 1,4-phenylene in which a hydrogen atom may be substituted with a halogen atom; and ring B is trans-1,4-cyclohexylene or 1,4-phenylene in which a hydrogen atom may be substituted with a halogen atom.

**11.** A liquid crystal composition which contains at least one liquid crystal compound as claimed in any one of claims 1 to 8 as a first component and at least one compound selected from the group consisting of the formulae (5) and (6) as a second component,

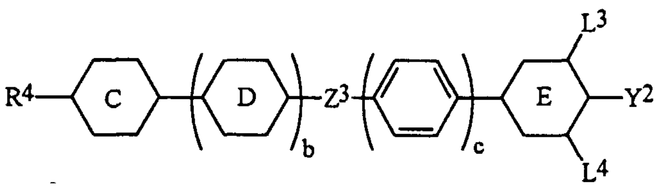

(5)

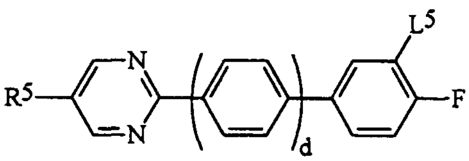

(6)

where $R^4$ and $R^5$ are each independently an alkyl group having 1 to 10 carbon atoms in which any unadjacent methylene group may be substituted with an oxygen atom or -CH=CH- and any hydrogen atom may be substituted with a fluorine atom; $Y^2$ is -CN or -C≡C-CN; ring C is trans-1,4-cyclohexylene, 1,4-phenylene, 1,3-dioxane-2,5-diyl or pyrimidine-2,5-diyl; ring D is trans-1,4-cyclohexylene, 1,4-phenylene in which a hydrogen atom may be substituted with a fluorine atom, or pyrimidine-2,5-diyl; ring E is trans-1,4-cyclohexylene or 1,4-phenylene; $Z^3$ is $-(CH_2)_2-$, -COO- or a single bond; $L^3$, $L^4$ and $L^5$ are each independently a hydrogen atom or a fluorine atom; and b, c and d are each independently 0 or 1.

**12.** A liquid crystal composition which contains at least one liquid crystal compound as claimed in any one of claims 1 to 8 as a first component and at least one compound selected from the group consisting of the formulae (7), (8) and (9) as a second component,

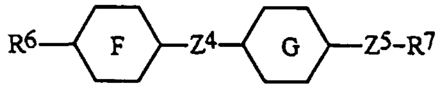
(7)

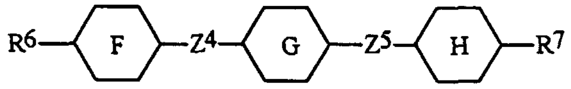
(8)

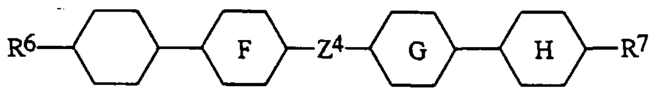
(9)

where $R^6$ and $R^7$ are each independently an alkyl group having 1 to 10 carbon atoms in which any unadjacent methylene group may be substituted with an oxygen atom or -CH=CH- and any hydrogen atom may be substituted with a fluorine atom; rings F, G and H are each independently trans-1,4-cyclohexylene, pyrimidine-2,5-diyl or 1,4-phenylene in which a hydrogen atom may be substituted with a fluorine atom; and $Z^4$ and $Z^5$ are each independently -C≡C-, -COO-, $-(CH_2)_2-$, -CH=CH- or a single bond.

**13.** A liquid crystal composition which contains at least one liquid crystal compound as claimed in any one of claims 1 to 8 as a first component and at least one compound selected from the group consisting of the formulae (10), (11) and (12) as a second component,

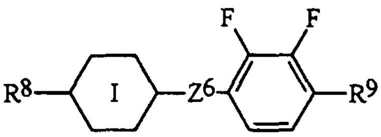
(10)

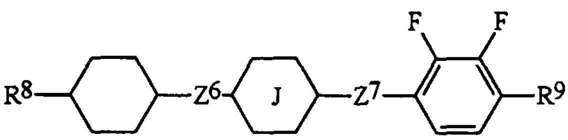
(11)

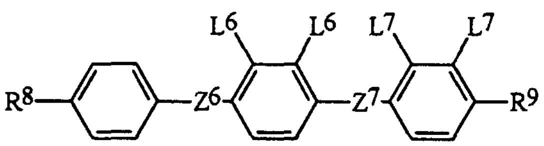
(12)

wherein $R^6$ and $R^9$ are each independently an alkyl group having 1 to 10 carbon atoms or an alkenyl group having 2 to 10 carbon atoms in which any methylene group ($-CH_2-$) may be substituted with an oxygen atom (-O-) but two methylene groups are not continuously substituted with oxygen atoms and any hydrogen atom in the alkyl group may be substituted with a fluorine atom; ring I and ring J are each independently a trans-1,4-cyclohexylene group or a 1,4-phenylene group; $L^6$ and $L^7$ are each independently a hydrogen atom or a fluorine atom with the proviso

that $L^6$ and $L^7$ cannot be hydrogen atoms simultaneously; and $Z^6$ and $Z^7$ are each independently $-(CH_2)_2-$, $-CH_2O-$ or a single bond.

**14.** A liquid crystal composition which comprises at least one liquid crystal compound as claimed in any one of claims 1 to 8 as a first component, at least one compound selected from the group consisting of the formulae (2), (3) and (4) as a second component and at least one compound selected from the group consisting of the formulae (5) and (6) as a third component.

**15.** A liquid crystal composition which comprises at least one liquid crystal compound as claimed in any one of claims 1 to 8 as a first component, at least one compound selected from the group consisting of the formulae (2), (3) and (4) as a second component and at least one compound selected from the group consisting of the formulae (7), (8) and (9) as a third component.

**16.** A liquid crystal composition which comprises at least one liquid crystal compound as claimed in any one of claims 1 to 8 as a first component, at least one compound selected from the group consisting of the formulae (5) and (6) as a second component and at least one compound selected from the group consisting of the formulae (7), (8) and (9) as a third component.

**17.** A liquid crystal composition which comprises at least one liquid crystal compound as claimed in any one of claims 1 to 8 as a first component, at least one compound selected from the group consisting of the formulae (2), (3) and (4) as a second component, at least one compound selected from the group consisting of the formulae (5) and (6) as a third component and at least one compound selected from the group consisting of the formulae (7), (8) and (9) as a fourth component.

**18.** A liquid crystal composition which contains at least one liquid crystal composition as claimed in any one of claims 9 to 17 and at least one optically active compound.

**19.** A liquid crystal display device which comprises a liquid crystal composition as claimed in any one of claims 9 to 18.

European Patent Office

## PARTIAL EUROPEAN SEARCH REPORT

which under Rule 45 of the European Patent Convention shall be considered, for the purposes of subsequent proceedings, as the European search report

Application Number EP 99 12 5327

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DATABASE WPI<br>Section Ch, Week 198104<br>Derwent Publications Ltd., London, GB;<br>Class E13, AN 1981-04959D<br>XP002134544<br>& JP 55 149372 A (HITACHI LTD),<br>20 November 1980 (1980-11-20)<br>* abstract * | 1,7-9,19 | C07D307/36<br>C09K19/34<br>C09K19/42<br>C09K19/46 |
| D,A | DATABASE WPI<br>Section Ch, Week 198817<br>Derwent Publications Ltd., London, GB;<br>Class E13, AN 1988-115423<br>XP002134545<br>& JP 63 060981 A (KAWASAKI KASEI KOGYO KK)<br>, 17 March 1988 (1988-03-17)<br>* abstract * | 1 | |
| A | MOLECULAR CRYSTALS AND LIQUID CRYSTALS ,<br>vol. 131, no. 3/4, 1985, pages 257-271,<br>XP002134543<br>GORDON AND BREACH SCIENCE PUBLISHERS,<br>READING., GB<br>* the whole document * | 1 | TECHNICAL FIELDS SEARCHED (Int.Cl.7)<br>C07D<br>C09K |
| | -/-- | | |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 31 March 2000 | Puetz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 99 12 5327

| Category | Citation of document with indication, where appropriate, of relevant passages | Relevant to claim | CLASSIFICATION OF THE APPLICATION (Int.Cl.7) |
|---|---|---|---|
| A | DE 44 46 836 A (HOECHST AG) 29 June 1995 (1995-06-29) * claims 1,2,4,5; example 5 * | 1,9,19 | |
| A | SHENG, HUAIYU ET AL: "Palladium-catalyzed rearrangement of 2-alkynyl aryl ketones to substitute furans" SYNTHESIS (1987), (11), 1022-3 ,1987, XP000891607 * page 1023, compound 3c * | 1,2,5,7 | |
| A | SHENG, HUAIYU ET AL: "A novel palladium-catalyzed rearrangement of acetylenic ketones to furans" TETRAHEDRON LETT. (1986), 27(40), 4893-4 , 1986, XP000891616 * the whole document * | 1,7,8 | TECHNICAL FIELDS SEARCHED (Int.Cl.7) |
| A | GALLARDO H ET AL: "NEW MESOGENIC THIOPHENE AND FURAN DERIVATIVES" LIQUID CRYSTALS,GB,TAYLOR AND FRANCIS LTD, LONDON, vol. 13, no. 1, 1 January 1993 (1993-01-01), pages 115-125, XP000343329 ISSN: 0267-8292 * page 116 - page 119 * | 1,7,8 | |
| A | WO 96 15126 A (UNIV GEORGIA ;UNIV NORTH CAROLINA (US); UNIV AUBURN (US); BOYKIN D) 23 May 1996 (1996-05-23) * examples 1,22 * | 1,2,5,7 | |
| A | WO 96 38412 A (TSUMURA & CO ;TAKEDA MASAKAZU (JP); YAMAZAKI YUKO (JP); KIGAWA MAS) 5 December 1996 (1996-12-05) * the whole document * | 1 | |

EPO FORM 1503 03.82 (P04C10)

European Patent Office

**INCOMPLETE SEARCH SHEET C**

Application Number
EP 99 12 5327

Claim(s) searched incompletely:
1-19

Reason for the limitation of the search:

Present claims 1-19 relate to an extremely large number of possible liquid crystal compounds, liquid crystal compositions comprising the claimed compounds and liquid crystal display devices comprising the liquid crystal compositions. Support within the meaning of Article 84 EPC and disclosure within the meaning of Article 83 EPC is to be found, however, for only a very small proportion of the compounds, compositions and display devices claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds and compositions recited in the examples (compounds prepared in the examples 1-5 and a reasonable generalisation thereof and compositions of examples 6-21).

## ANNEX TO THE EUROPEAN SEARCH REPORT ON EUROPEAN PATENT APPLICATION NO. EP 99 12 5327

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2000

| Patent document cited in search report | Publication date | Patent family member(s) | Publication date |
|---|---|---|---|
| JP 55149372 A | 20-11-1980 | JP 1325872 C | 16-07-1986 |
| | | JP 60050234 B | 07-11-1985 |
| JP 63060981 A | 17-03-1988 | NONE | |
| DE 4446836 A | 29-06-1995 | JP 7188661 A | 25-07-1995 |
| | | US 5853612 A | 29-12-1998 |
| WO 9615126 A | 23-05-1996 | AU 692024 B | 28-05-1998 |
| | | AU 4283896 A | 06-06-1996 |
| | | CA 2204898 A | 23-05-1996 |
| | | EP 0792271 A | 03-09-1997 |
| | | JP 10508857 T | 02-09-1998 |
| | | US 5602172 A | 11-02-1997 |
| | | ZA 9509661 A | 29-05-1996 |
| WO 9638412 A | 05-12-1996 | JP 11228535 A | 24-08-1999 |

EPO FORM P0459

For more details about this annex : see Official Journal of the European Patent Office, No. 12/82